# EUROPÄISCHE PATENTSCHRIFT

(11) **EP 4 551 280 B1**
(45) Veröffentlichungstag und Bekanntmachung des Hinweises auf die Patenterteilung: **09.09.2026**
(21) Anmeldenummer: 23740967.7
(22) Anmeldetag: 05.07.2023
(51) Int. Cl.: A61M 16/00

(54) **BEATMUNGSGERÄT ZUR ATEMGASVERSORGUNG**
VENTILATOR FOR SUPPLYING RESPIRATORY GAS
VENTILATEUR MÉCANIQUE POUR L'ALIMENTATION EN GAZ RESPIRATOIRE

(30) Priorität: 08.07.2022 DE 102022117141
(43) Veröffentlichungstag der Anmeldung: 14.05.2025
(73) Patentinhaber: Rheinisch-Westfälische Technische Hochschule Aachen, abgekürzt RWTH Aachen, Körperschaft des öffentlichen Rechts, 52062 Aachen (DE); Löwenstein Medical Technology SA, 2557 Luxemburg (LU)
(72) Erfinder: BUGLOWSKI, Mateusz, 52076 Aachen (DE); PFANNSCHMIDT, Valerie, 52074 Aachen (DE); STOLLENWERK, André, 52072 Aachen (DE); SCHOBERER, Mark, 52062 Aachen (DE); NGUYEN, Thi Bich Huong, 52062 Aachen (DE); BRAUN, Oliver, 56130 Bad Ems (DE); BECKER, Sabine, 52062 Aachen (DE)
(74) Vertreter: Eisenführ Speiser
(86) Internationale Anmeldenummer: PCT/EP2023/068525
(87) Internationale Veröffentlichungsnummer: WO 2024/008785

(56) Entgegenhaltungen:
- EP-A1- 1 985 326
- WO-A1-00/44427
- DE-A1- 102021 000 313

## Beschreibung

Die Erfindung betrifft ein Beatmungsgerät zur Atemgasversorgung, eine Steuereinrichtung zur Ansteuerung einer Atemgasquelle eines Beatmungsgeräts, ein Verfahren zur Atemgasversorgung und ein Computerprogramm.

Beatmungsgeräte zur Atemgasversorgung sind im Allgemeinen bekannt als lebensrettendes Mittel. Sie bergen aber auch verschiedene Risiken für den zu beatmenden Patienten, insbesondere, wenn falsche Einstellungen des Beatmungsgeräts vorgenommen werden.

In diesem Zusammenhang ist bekannt, Einstellungen der Beatmungsgeräte abhängig von Patientendaten oder einem Zustand des zu beatmenden Patienten vorzunehmen. Es ist aber auch wichtig, eine Anpassung während des Beatmungszeitraums vorzunehmen, um zum Beispiel die Entwicklung des Zustands des zu beatmenden Patienten zu berücksichtigen und insbesondere auf Spontanatmung zu reagieren. Beispielsweise wird dafür bekanntermaßen der arterielle CO2-Partialdruck einbezogen, der mit Hilfe einer Blutprobe oder mit Hilfe einer Elektrode auf der Haut bestimmt werden kann. Jedoch ist beides insbesondere bei Säuglingen nicht unproblematisch, da etwa ein Bestimmen mit Hilfe einer Elektrode mit einer gewissen Temperatur betrieben werden muss, und dies Verbrennungsschäden auf der Haut erzeugen kann. Alternativ wird deshalb der arterielle CO2-Partialdruck teilweise basierend auf einem CO2-Gehalt der ausgeatmeten Luft (sogenannter endtidaler CO2-Partialdruck) ermittelt, wobei in diesem Fall eine höhere Fehlertoleranz in Kauf genommen wird. Auf Basis dieser Daten können dann Einstellungen des Beatmungsgeräts vorgenommen werden.

Eine Einstellung des Beatmungsgeräts verläuft üblicherweise über eine Einstellung einer Atemfrequenz und eines maximalen Inspirationsdrucks, wobei eine geschlossene Feedbackkontrolle zur Regelung der Einstellungen vorgesehen werden kann. Jedoch ist eine genaue Regelung dieser Werte notwendig, um das Risiko für Schäden durch die Beatmung, zum Beispiel durch einen zu hohen Druck oder eine zu hohe Atemfrequenz, zu minimieren, die insbesondere an der Lunge entstehen können. Eine ungenaue Regelung des Beatmungsgeräts kann außerdem zu einem zu niedrigen CO2-Gehalt im Blut führen, was ein Risiko für Schäden insbesondere des Gehirns bergen kann. Vor allem bei Kindern, Neugeborenen und besonders bei Frühgeborenen haben bereits kleine Abweichungen von einer optimalen Beatmung ein hohes Risiko für Schäden. Bei Neugeborenen kommt zudem hinzu, dass sich die Lunge über den zu beatmenden Zeitraum, der sich über mehrere Stunden bis Tage erstrecken kann, stark verändert, wodurch eine kontinuierliche Anpassung notwendig ist, um eine optimale Beatmung bereitzustellen.

Aus dem Stand der Technik sind beispielsweise Beatmungsgeräte aus US 7,802,571 B2 und EP 1 984 050 B1 bekannt.

Es ist somit Aufgabe der vorliegenden Erfindung, eine Lösung vorzusehen, die die oben genannten Nachteile des Standes der Technik ausgleicht und eine Sicherheit von Beatmungsgeräten verbessert.

Nach einem ersten Aspekt wird ein Beatmungsgerät vorgeschlagen, zur Atemgasversorgung, insbesondere von Neugeborenen. Es umfasst vorzugsweise einige oder alle der folgenden Elemente: eine Atemgasquelle, eine Steuereinrichtung zur Ansteuerung der Atemgasquelle, eine mit der Steuereinrichtung verbundene Sensoreinrichtung zum Erfassen eines endtidalen CO2-Partialdrucks, einen auswechselbaren Atemgasschlauch mit zumindest einem ersten Anschlussstutzen für den Atemgasschlauch an der Atemgasquelle und einem zweiten Anschlussstutzen für den Atemgasschlauch an einem Patienteninterface, wie beispielsweise einem Mundstück, und eine Nutzerschnittstelle, die ausgestaltet ist, Nutzereingaben zu empfangen. Die Steuereinrichtung umfasst vorzugsweise eine Zielwertbereitstellungseinheit, die ausgestaltet ist, einen Zielwert des arteriellen CO2-Partialdrucks bereitzustellen. Die Steuereinrichtung umfasst vorzugsweise eine Minutenvolumenermittlungseinheit, die ausgestaltet ist, basierend auf dem Zielwert des arteriellen CO2-Partialdrucks und einem ermittelten Wert für den arteriellen CO2-Partialdruck oder einem aus dem endtidalen CO2-Partialdruck abgeleiteten Wert für den arteriellen CO2-Partialdruck einen Zielwert eines Minutenvolumens zu ermitteln, falls der ermittelte Wert für den arteriellen CO2-Partialdruck oder der aus dem endtidalen CO2-Partialdruck abgeleitete Wert für den arteriellen CO2-Partialdruck außerhalb eines ersten vordefinierten Wertebereichs um den Zielwert des arteriellen CO2-Partialdrucks liegt oder über einen Zeitraum kleiner als einem vordefinierten Zeitraum in dem ersten vordefinierten Wertebereich liegt. Die Steuereinrichtung umfasst ferner vorzugsweise eine Atemgasquellesteuereinheit, die ausgestaltet ist, den Zielwert des Minutenvolumens zu empfangen und die Atemgasquelle, vorzugsweise den Ventilator, basierend auf dem Zielwert des Minutenvolumens anzusteuern. Die Atemgasquelle kann zumindest ein Motor und/oder Ventilator, ein Ventil, einen Balg, eine Membran, eine Druckgasvorrichtung sein oder umfassen oder eine Kombination der vorgenannten. Wenn in der Folge beispielhaft ein Ventilator genannt ist, soll verstanden werden, dass auch die anderen Varianten umfasst sind.

Wenn im Folgenden von CO2-Partialdruck ohne weitere Zusätze bzw. von einem Zielwert des CO2-Partialdrucks gesprochen wird, ist hiermit bevorzugt der arterielle CO2-Partialdruck angesprochen. Dieser Ausdruck bezieht sich auf den Ort des Zielwerts. Ein Eingriff in den Patienten ist hierfür nicht erforderlich und die Erfindung bezieht sich lediglich auf die Vorrichtung sowie das Steuerverfahren innerhalb der Vorrichtung.

Die Sensoreinrichtung zum Erfassen des endtidalen CO2-Partialdrucks umfasst vorzugsweise einen Sensor zum direkten und/oder indirekten Erfassen des endtidalen CO2-Partialdrucks, wobei die Sensoreinrichtung beim indirekten Erfassen des endtidalen CO2-Partialdrucks eine Messgröße erfasst, aus der der endtidale CO2-Partialdruck berechnet werden kann. Die Sensoreinrichtung ist vorzugsweise ausgestaltet, den erfassten und/oder berechneten CO2-Partialdruck auszugeben, bevorzugt an die Minutenvolumenermittlungseinheit oder einen Zwischenspeicher.

Ein Sensor kann direkt in dem Mundstück oder in der Nähe des Mundstücks, zum Beispiel im Atemgasschlauch oder einem Verbindungsstück zum Mundstück bzw. Atemgasschlauch, angeordnet sein, wobei der Sensor so positioniert ist, dass ein Erfassen des endtidalen CO2-Partialdrucks in einem Atemgas möglich ist. Ein Mundstück kann zum Beispiel ein Tubus oder eine Atemmaske sein. Das Mundstück, d.h. der Tubus bzw. die Atemmaske weisen einen Atemgasausgang auf, durch den Atemgas zum Beispiel in eine Lunge eines Patienten geleitet werden kann. Bevorzugt ist die Sensoreinrichtung, insbesondere der Sensor, so angeordnet, dass der endtidale CO2-Partialdruck zwischen Atemgasquelle und Atemgasausgang erfasst wird, bevorzugt zwischen Atemgasschlauch und Atemgasausgang, besonders bevorzugt möglichst nahe an der Lunge des Patienten. Besonders bevorzugt ist der Sensoreinrichtung bzw. der Sensor unabhängig einer Wahl des Mundstücks.

Die Nutzerschnittstelle ist vorzugsweise eine Eingabeeinheit, die vorzugsweise einen Touch-Bildschirm und/oder Knöpfe oder Schalter umfasst. Alternativ oder zusätzlich kann die Nutzerschnittstelle auch einen Computer mit einem Bildschirm, einer Maus und/oder einer Tastatur umfassen. Die Nutzerschnittstelle kann durch ein Kabel oder kabellos mit den weiteren Komponenten, beispielsweise der Steuereinrichtung, verbunden sein. Falls eine kabellose Verbindung zwischen Nutzerschnittstelle und Steuereinrichtung vorliegt, kann die Nutzerschnittstelle vorzugsweise auch ein Tablet, Smartphone etc. umfassen. Die Nutzerschnittstelle kann eine grafische Benutzeroberfläche (GUI) anzeigen, mit der ein Nutzer Einstellungen vordefinieren, ändern und/oder überwachen kann.

Die Steuereinrichtung umfasst vorzugsweise eine Zielbereitstellungseinheit, die ausgestaltet ist, einen Zielwert des arteriellen CO2-Partialdrucks bereitzustellen. Der Zielwert des arteriellen CO2-Partialdrucks entspricht dem Wert des arteriellen CO2-Partialdrucks, der während der Beatmung erreicht oder gehalten werden soll. Der Zielwert des arteriellen CO2-Partialdrucks ist ein vordefinierter Wert, der einem Standardwert des arteriellen CO2-Partialdrucks entsprechen kann. Bevorzugt basiert der Zielwert des arteriellen CO2-Partialdrucks auf Erfahrungswerten des Nutzers. Zusätzlich oder alternativ kann der Zielwert des arteriellen CO2-Partialdrucks anhand von Patientendaten und/oder basierend auf Messdaten bestimmt werden. Bevorzugt ist der Zielwert des arteriellen CO2-Partialdrucks größer als 35 mmHg, besonders bevorzugt 40 mmHg, und kleiner als 45 mmHg.

Die Minutenvolumenermittlungseinheit ist vorzugsweise ausgestaltet, einen Zielwert eines Minutenvolumens zu ermitteln. Das Minutenvolumen entspricht einem Volumen an Atemluft, das pro Minute von einem Patienten ein- und wieder ausgeatmet wird. Der Zielwert des Minutenvolumens entspricht dem Wert des Minutenvolumens, der durch das Beatmungsgerät bereitgestellt werden soll. Der Zielwert des Minutenvolumens wird basierend auf dem Zielwert des arteriellen CO2-Partialdrucks und einem ermittelten Wert für den arteriellen CO2-Partialdruck und/oder einem aus dem endtidalen CO2-Partialdruck abgeleiteten Wert für den arteriellen CO2-Partialdruck ermittelt. Ein ermittelter Wert für den arteriellen CO2-Partialdruck kann zum Beispiel aus einer bereitgestellten Blutprobe eines Patienten ermittelt werden. Der aus dem endtidalen CO2-Partialdruck abgeleiteten Wert für den arteriellen CO2-Partialdruck ist ein arterieller CO2-Partialdruck, der aus dem endtidalen CO2-Partialdruck abgeleitet wird. Insbesondere wird der arterielle CO2-Partialdruck durch den endtidalen CO2-Partialdruck abgeschätzt bzw. genähert.

In einem Aspekt der Erfindung wird ein Minutenvolumen lediglich dann ermittelt, falls der ermittelte Wert für den arteriellen CO2-Partialdruck oder der aus dem endtidalen CO2-Partialdruck abgeleitete Wert für den arteriellen CO2-Partialdruck außerhalb eines ersten vordefinierten Wertebereichs um den Zielwert des arteriellen CO2-Partialdrucks liegt und/oder über einen Zeitraum kleiner als einem vordefinierten Zeitraum in dem ersten vordefinierten Wertebereich liegt. Der vordefinierte Zeitraum sollte ausreichend lang sein, um sicherzustellen, dass der arterielle CO2-Partialdruck "ausgeregelt" ist und sich nicht mehr im transienten Bereich befindet. Der vordefinierte Zeitraum wird durch einen Nutzer vordefiniert, zum Beispiel durch Eingabe eines Zeitraums, vorzugsweise mittels der Nutzerschnittstelle, oder basiert auf voreingestellten Werten, die zum Beispiel aus einem Speicher eingelesen werden können. In einer bevorzugten Ausführungsform ist der vordefinierte Zeitraum 60 s bis 100 s. Bevorzugt liegt der Zielwert des arteriellen CO2-Partialdrucks in einer Mitte des ersten vordefinierten Wertebereichs. Der erste vordefinierte Wertebereich kann durch eine Größe 2w angegeben werden, wobei im Fall, dass der Zielwert des arteriellen CO2-Partialdrucks in der Mitte des ersten vordefinierten Wertebereichs liegt, eine Größe w einer Abweichung von dem Zielwert des arteriellen CO2-Partialdrucks entspricht. Der erste vordefinierte Wertebereich kann beispielsweise (und bevorzugt) durch w = 2 mmHg gegeben sein.

Die Atemgasquellesteuereinheit ist vorzugsweise ausgestaltet, den Zielwert des Minutenvolumens zu empfangen und die Atemgasquelle, vorzugsweise den Ventilator, basierend auf dem Zielwert des Minutenvolumens anzusteuern.

Das Beatmungsgerät gemäß einem Aspekt der Erfindung passt das Minutenvolumen vorzugsweise nur in den Fällen an, in denen die oben genannte Bedingung erfüllt ist, d.h. das Minutenvolumen wird vorzugsweise nicht angepasst, wenn die oben genannte Bedingung nicht erfüllt ist. Dies führt dazu, dass ein häufiges Anpassen des Minutenvolumens verhindert wird und ein Anpassen des Minutenvolumens nur dann stattfindet, wenn der ermittelte Wert für den arteriellen CO2-Partialdruck oder der aus dem endtidalen CO2-Partialdruck abgeleitete Wert für den arteriellen CO2-Partialdruck außerhalb des ersten vordefinierten Wertebereichs liegt und/oder über einen Zeitraum kleiner als einem vordefinierten Zeitraum in dem ersten vordefinierten Wertebereich liegt. Dadurch kann eine schonendere Beatmung durch Reduktion der Anzahl an Stelleingriffen bei gleichzeitig unbeeinflusstem Einpendeln des arteriellen CO2-Partialdrucks um den Zielwert ermöglicht werden. Dadurch kann eine Sicherheit des Beatmungsgeräts erhöht werden.

Insbesondere kann durch das erfindungsgemäße Beatmungsgerät eine automatische und kontinuierliche Anpassung des Beatmungsgeräts an den Patienten bereitgestellt werden.

Die Minutenvolumenermittlungseinheit kann durch einen geeigneten Regler, insbesondere einen PI-Regler, realisiert werden, der basierend auf den oben genannten Merkmalen einen Zielwert, insbesondere einen weiteren Zielwert, für das Minutenvolumen ermittelt. "Weiterer Zielwert" bedeutet hier, dass bereits ein Minutenvolumen eingestellt ist, dass also das Beatmungsgerät basierend auf einem Zielwert für das Minutenvolumen betrieben wird, und dass die Minutenvolumenermittlungseinheit ausgestaltet ist, einen weiteren Zielwert für das Minutenvolumen zu ermitteln. Die Minutenvolumenermittlungseinheit gibt den weiteren Zielwert für das Minutenvolumen an die Atemgasquellesteuereinheit weiter. Die Atemgasquellesteuereinheit ist ausgestaltet, den weiteren Zielwert des Minutenvolumens zu empfangen und die Atemgasquelle, vorzugsweise den Ventilator, basierend auf dem weiteren Zielwert für das Minutenvolumen anzusteuern. Dadurch kommt es zu einer Veränderung in der Ansteuerung der Atemgasquelle, vorzugsweise des Ventilators, d.h. zu einem Stelleingriff.

Das beanspruchte Beatmungsgerät umfasst also vorzugsweise eine Steuereinrichtung, die einen geschlossenen Regelkreis zur Regelung des arteriellen CO2-Partialdrucks bei einer künstlichen Beatmung verwendet.

Jegliche Daten, d.h. insbesondere der Zielwert des arteriellen CO2-Partialdrucks, ein erster vordefinierter Wertebereich, ein zweiter vordefinierter Wertebereich, ein vordefinierter Zeitraum, Patientendaten etc., die für die Regelung des arteriellen CO2-Partialdrucks bei künstlicher Beatmung durch das erfindungsgemäße Beatmungsgerät verwendet werden, können über einen Speicher bereitgestellt werden oder über eine Schnittstelle, beispielsweise der Nutzerschnittstelle, empfangen werden. Patientendaten können zum Beispiel von einem patientenindividuellen Speicher, etwa einer Chipkarte oder einem Speicher in einer Cloud, ausgelesen werden und/oder beispielsweise manuell, d.h. über die Nutzerschnittstelle durch einen Nutzer, eingegeben werden oder über eine Verbindung, insbesondere eine kabellose Verbindung, an der Steuerungseinrichtung bereitgestellt werden.

In einer vorteilhaften Ausgestaltung eines Aspekts der Erfindung ist die Atemgasquellesteuereinheit ferner ausgestaltet, einen voreingestellten Wert für das Minutenvolumen zu empfangen und die Atemgasquelle, vorzugsweise den Ventilator, basierend auf dem voreingestellten Wert des Minutenvolumens anzusteuern, falls der ermittelte arterielle CO2-Partialdruck oder der von dem endtidalen CO2-Partialdruck abgeleitete Wert für den arteriellen CO2-Partialdruck über den vordefinierten Zeitraum in dem ersten vordefinierten Wertebereich und nach dem vordefinierten Zeitraum in einem zweiten vordefinierten Wertebereich um den Zielwert des arteriellen CO2-Partialdrucks liegt, wobei der zweite vordefinierte Wertebereich vorzugsweise größer ist als der erste vordefinierte Wertebereich.

Es ist vorteilhaft, ein Minutenvolumen lediglich dann zu ermitteln, falls der ermittelte Wert für den arteriellen CO2-Partialdruck oder der aus dem endtidalen CO2-Partialdruck abgeleitete Wert für den arteriellen CO2-Partialdruck außerhalb eines ersten vordefinierten Wertebereichs liegt oder über einen Zeitraum, der kleiner ist als der vordefinierte Zeitraum, innerhalb des ersten vordefinierten Wertebereichs liegt. Vorzugsweise wird ein Minutenvolumen nicht ermittelt, falls der ermittelte arterielle CO2-Partialdruck oder der aus dem endtidalen CO2-Partialdruck abgeleitete Wert für den arteriellen CO2-Partialdruck über den vordefinierten Zeitraum innerhalb des ersten vordefinierten Wertebereichs und nach Ende des vordefinierten Zeitraums innerhalb eines zweiten vordefinierten Wertebereichs liegt, der um den Zielwert des arteriellen CO2-Partialdrucks vorgesehen ist. Der Term "nach dem vordefinierten Zeitraum" bezieht sich auf einen Zeitraum, der zeitlich nach dem vordefinierten Zeitraum liegt, wobei es bevorzugt ist, dass der ermittelte Wert für den arteriellen CO2-Partialdruck oder der aus dem endtidalen CO2-Partialdruck abgeleitete Wert für den arteriellen CO2-Partialdruck unmittelbar nach dem vordefinierten Zeitraum in dem zweiten vordefinierten Wertebereich liegt. Das Wort "in" in Bezug auf den ersten oder zweiten vordefinierten Wertebereich bezieht sich darauf, dass der ermittelte Wert für den arteriellen CO2-Partialdruck oder der aus dem endtidalen CO2-Partialdruck abgeleitete Wert für den arteriellen CO2-Partialdruck innerhalb des vordefinierten Wertebereichs, d.h. innerhalb vordefinierter Grenzen, liegt. Bevorzugt liegt der Zielwert des arteriellen CO2-Partialdrucks in einer Mitte des zweiten vordefinierten Wertebereichs. Wie bereits oben angegeben, liegt der Zielwert des arteriellen CO2-Partialdrucks bevorzugt in einer Mitte des ersten vordefinierten Wertebereichs. Es ist dabei besonders bevorzugt, dass der Zielwert des arteriellen CO2-Partialdrucks in einer Mitte des ersten vordefinierten Wertebereichs und einer Mitte des zweiten vordefinierten Wertebereichs liegt. Der zweite vordefinierte Wertebereich kann durch eine Größe 2L angegeben werden, wobei im Fall, dass der Zielwert des arteriellen CO2-Partialdrucks in der Mitte des zweiten vordefinierten Wertebereichs liegt, eine Größe L einer Abweichung von dem Zielwert entspricht. Der zweite vordefinierte Wertebereich ist vorzugsweise größer als der erste vordefinierte Wertebereich, d.h. es gilt vorzugsweise L>w. Bevorzugt ist L= 6mmHg.

Das führt dazu, dass ein kleinerer Wertebereich als Bedingung erfüllt sein muss, wenn der arterielle CO2-Partialdruck weit von dem Zielwert des arteriellen CO2-Partialdrucks, d.h. außerhalb des ersten vordefinierten Wertebereichs, liegt und ein größerer Wertebereich als Bedingung erfüllt sein muss, wenn der arterielle CO2-Partialdruck innerhalb des ersten vordefinierten Wertebereichs liegt. Die bevorzugte Ausführung führt somit zu einer weiteren Reduzierung der Stelleingriffe und kann damit zu einer Verbesserung der Sicherheit des Beatmungsgeräts beitragen.

In einer anderen vorteilhaften Ausgestaltung eines Aspekts der Erfindung ist die Minutenvolumenermittlungseinheit ausgestaltet, basierend auf dem Zielwert des arteriellen CO2-Partialdrucks und einem von dem endtidalen CO2-Partialdruck abgeleiteten Wert den Zielwert für das Minutenvolumen zu ermitteln, wobei der abgeleitete Wert einer abschnittsweise linearen Näherung auf Basis des endtidalen CO2-Partialdrucks entspricht.

Es konnte gefunden werden, dass zu einem Zeitpunkt der arterielle CO2-Partialdruck auf Basis des endtidalen CO2-Partialdrucks über einen linearen Zusammenhang angenähert werden kann, d.h. dass zu einem Zeitpunkt der endtidale CO2-Partialdruck ermittelt wird und dieser über einen linearen Zusammenhang angenähert wird. Über die Zeit führt dies zu einer abschnittsweisen, d.h. teilweise, linearen Näherung.

In einer bevorzugten Variante der obigen Ausgestaltung wird der von dem endtidalen CO2-Partialdruck abgeleitete Wert durch folgende Gleichung berechnet:$PaCO 2 = C 1 \times PetCO 2 + C 2 ,$ wobei Pa^CO2 der von dem endtidalen CO2-Partialdrucks abgeleitete Wert ist, PetCO2 der endtidale CO2-Partialdruck und C1 und C2 vordefinierte Parameter. Bevorzugt können die vordefinierten Parameter C1 und C2 abhängig von Patientendaten ermittelt werden und/oder basierend auf Erfahrungswerten, zum Beispiel eines Arztes, gesetzt, d.h. voreingestellt, werden. Bevorzugt können für die vordefinierten Parameter C1 und C2 Initialwerte aus einer bereitgestellten Blutprobe ermittelt werden, die dann, zum Beispiel mit Hilfe eines Algorithmus, weiter angepasst werden können. In einer Ausführungsform kann C1=1 sein oder C1 ungleich 1 sein, wobei dann bevorzugt ist, dass C1 größer als 0,9 und kleiner als 1,1 ist. Alternativ kann auch angenommen werden, dass der endtidale CO2-Partialdruck dem arteriellen CO2-Partialdruck entspricht. Beispielsweise kann C2 =6,65 mmHg sein, wie auch bekannt aus Bhat, Y. R.; Abhishek, N.: Mainstream endtidal carbon dioxide monitoring in ventilated neonates. In: Singapore Medical Journal 49, (2008), March, Nr. 3, S. 199-203. Eine Möglichkeit zur Bestimmung von C2 kann zum Beispiel auch der US 7 902 571 entnommen werden. Alternativ zu einer linearen Näherung kann auch eine Näherung vorgenommen werden, die eine Temperatur und/oder eine Feuchtigkeit, bevorzugt in dem Mundstück, berücksichtigt.

In einer weiteren vorteilhaften Ausgestaltung eines Aspekts der Erfindung ist die Atemgasquellesteuereinheit ausgestaltet, basierend auf dem ermittelten Zielwert des Minutenvolumens einen ersten maximalen Inspirationsdruck und eine erste Atemfrequenz zum Ansteuern der Atemgasquelle, vorzugsweise des Ventilators, zu ermitteln, und die Atemgasquelle, vorzugsweise den Ventilator, basierend auf dem ersten ermittelten maximalen Inspirationsdruck und der ersten ermittelten Atemfrequenz anzusteuern.

Der maximale Inspirationsdruck entspricht einem maximalen Druck, der durch das Beatmungsgerät während einer Inspiration erzeugt wird. Die Atemfrequenz gibt die Anzahl der Atemzüge innerhalb einer bestimmten Zeitspanne an.

In einer bevorzugten Variante ist die Atemgasquellesteuereinheit ferner ausgestaltet, ein gemessenes Minutenvolumen, den ersten ermittelten maximalen Inspirationsdruck und/oder die erste ermittelte Atemfrequenz zu empfangen, basierend zumindest auf dem gemessenen Minutenvolumen, dem ersten ermittelten maximalen Inspirationsdruck und/oder der ersten ermittelten Atemfrequenz einen zweiten maximalen Inspirationsdruck und eine zweite Atemfrequenz zu ermitteln, und den die Atemgasquelle, vorzugsweise Ventilator, basierend auf dem zweiten ermittelten maximalen Inspirationsdruck und der zweiten ermittelten Atemfrequenz anzusteuern.

Das gemessene Minutenvolumen kann dabei als ein Messwert oder als ein von einem Messwert abgeleiteter Wert des Minutenvolumens empfangen werden. Bevorzugt kann das Minutenvolumen über eine kontinuierliche Atemgasflussmessung bestimmt werden, wobei aus dem kontinuierlichen Atemgasfluss das Minutenvolumen ermittelt werden kann. Bevorzugt wird ein maximaler Inspirationsdruck mit einer Schrittweite von 1 mbar und eine Atemfrequenz mit einer Schrittweite von 2/min angepasst, d.h. ein erster maximaler Inspirationsdruck hat zu einem zweiten maximalen Inspirationsdruck eine Differenz von bevorzugt 1 mbar und eine erste Atemfrequenz hat zu einer zweiten Atemfrequenz eine Differenz von bevorzugt 2/min. Alternativ kann ein maximaler Inspirationsdruck mit einer Schrittweite von 0,5 mbar und eine Atemfrequenz mit einer Schrittweite von 1/min gewählt werden, so dass eine langsamere Reaktion erfolgt. Es können aber auch andere Schrittweiten gewählt werden.

Insbesondere kann die beschriebene Regelung des arteriellen CO2-Partialdrucks als ein geschlossener Regelkreis verstanden werden, wobei der Regelkreis eine äußere Schleife aufweist, die der Regelung des arteriellen CO2-Partialdrucks durch das Minutenvolumen dient, und eine innere Schleife aufweist, durch die das Minutenvolumen durch den maximalen Inspirationsdruck und die Atemfrequenz eingestellt wird. Die äußere Schleife gibt somit einen Wert für die innere Schleife vor. Bevorzugt wird eine Ausführungsfrequenz des äußeren Regelkreises, d.h. die Frequenz, mit der der arterielle CO2-Partialdruck geregelt wird, von 1/20 Hz bis 1/60 Hz gewählt und ist besonders bevorzugt 1/20 Hz. Weiter wird bevorzugt eine Ausführungsfrequenz für den inneren Regelkreis, d.h. die Frequenz, mit der das Minutenvolumen geregelt wird, von 1/4 Hz bis 1/ Hz gewählt und ist besonders bevorzugt 1/4 Hz.

Eine derartige Regelung, insbesondere mit einem inneren Regelkreis und einem äußeren Regelkreis, ermöglicht ein weiteres Reduzieren von Stelleingriffen, wodurch die Sicherheit eines Patienten bei Verwenden des Beatmungsgeräts weiter erhöht werden kann.

In einer weiteren bevorzugten Variante ist die Atemgasquellesteuereinheit ferner ausgestaltet, einen PIP-Wertebereich mit einem unteren PIP-Grenzwert und einem oberen PIP-Grenzwert für den maximalen Inspirationsdruck, und/oder einem RR-Wertebereich mit einem unteren RR-Grenzwert und einem oberen RR-Grenzwert für die Atemfrequenz zu erhalten, und die Atemgasquelle, vorzugsweise den Ventilator, basierend auf dem ermittelten maximalen Inspirationsdruck und der ermittelten Atemfrequenz anzusteuern, falls der ermittelte maximale Inspirationsdruck in dem PIP-Wertebereich und/oder die ermittelte Atemfrequenz in dem RR-Wertebereich liegt.

Insbesondere kann dadurch erreicht werden, dass der maximale Inspirationsdruck und die Atemfrequenz zur Einstellung des Minutenvolumens nicht Werte annehmen können, die dem Patienten potenziell schädigen. Bevorzugt werden der untere PIP-Grenzwert, der obere PIP-Grenzwert, der untere RR-Grenzwert und/oder der obere RR-Grenzwert basierend auf Patientendaten, Erfahrungswerten, Richtlinien, und/oder einem Patientenzustand vordefiniert. In einer bevorzugten Ausführungsform sind der untere PIP-Grenzwert, der obere PIP-Grenzwert, der untere RR-Grenzwert und/oder der obere RR-Grenzwert in einem Speicher der Steuereinrichtung vordefiniert oder können durch die Nutzerschnittstelle eingegeben werden. Grenzwerte für die Eingabe bzw. Einstellung des maximalen Inspirationsdrucks und der Atemfrequenz ermöglichen eine Risikominimierung der Regelung des arteriellen CO2-Partialdrucks in Hinblick auf die Einstellung des maximalen Inspirationsdrucks und der Atemfrequenz.

In einer bevorzugten Weiterbildung ist die Atemgasquellesteuereinheit ferner ausgestaltet, Patientendaten, vorzugsweise eine Compliance und/oder einen Widerstand, zu empfangen, basierend zumindest auf dem ermittelten Minutenvolumen und den Patientendaten, einen patientenabhängigen maximalen Inspirationsdruck und eine patientenabhängige Atemfrequenz zu ermitteln, und die Atemgasquelle, vorzugsweise den Ventilator, basierend auf dem patientenabhängigen maximalen Inspirationsdruck und der patientenabhängigen Atemfrequenz anzusteuern.

Eine Compliance ist eine elastische Volumendehnbarkeit des Atemwegs, insbesondere der Lunge. Ein Widerstand kann als Atemwegswiderstand verstanden werden, den eine Atemluft bei ihrem Fluss durch die Atemwege, insbesondere der Lunge, überwinden muss.

Dadurch können, anstatt von standardisierten Werten für den maximalen Inspirationsdruck und die Atemfrequenz patientenspezifische Daten verwendet werden, was zu einer besonders vorteilhaften Regelung des arteriellen CO2-Partialdrucks führen kann.

In einer anderen bevorzugten Ausgestaltung ist die Atemgasquellesteuereinheit ferner ausgestaltet, einen Solltidalvolumenbereich Vt mit einem unteren Vt-Sollgrenzwert und einem oberen Vt-Sollgrenzwert zu empfangen, basierend zumindest auf dem ermittelten Minutenvolumen und dem Solltidalvolumenbereich Vt, einen tidalvolumenabhängigen maximalen Inspirationsdruck und eine tidalvolumenabhängige Atemfrequenz zu ermitteln, und die Atemgasquelle, vorzugsweise den Ventilator, basierend auf dem tidalvolumenabhängigen maximalen Inspirationsdruck und der tidalvolumenabhängigen Atemfrequenz anzusteuern.

In dieser Ausführungsform kann demnach ein Zielbereich für das Tidalvolumen für die Regelung des arteriellen CO2-Partialdrucks berücksichtigt werden, d.h. es wird ein Zielwert für das Minutenvolumen mit dem gewünschten Tidalvolumen, d.h. einem Tidalvolumen in dem Zielbereich für das Tidalvolumen, von der Minutenvolumenermittlungseinheit ermittelt, von der Atemgasquellesteuereinheit empfangen und die Atemgasquelle basierend auf diesem Zielwert für das Minutenvolumen angesteuert.

Bevorzugt handelt es sich bei dem unteren Vt-Sollgrenzwert und dem oberen Vt-Sollgrenzwert nicht um harte Grenzen, d.h. in bestimmten Situationen können der untere Vt-Sollgrenzwert und der obere Vt-Sollgrenzwert über- oder unterschritten werden. Somit wird priorisiert ein Minutenvolumen mit einem Tidalvolumen in dem Zielbereich für das Tidalvolumen bereitgestellt.

Die obige Variante kann vorteilhafterweise so ausgeführt sein, dass die Atemgasquellesteuereinheit ferner ausgestaltet ist, falls ein gemessenes Tidalvolumen nicht im Solltidalvolumenbereich Vt liegt, basierend zumindest auf dem ermittelten Minutenvolumen einen zweiten tidalvolumenabhängigen maximalen Inspirationsdruck zu ermitteln und die Atemgasquelle basierend auf dem zweiten tidalvolumenabhängigen maximalen Inspirationsdruck anzusteuern.

Das gemessene Tidalvolumen kann ein Messwert sein, oder ein von einem Messwert abgeleiteten Wert. Beispielsweise kann ein Tidalvolumen über eine Messung des Atemwegsflusses berechnet werden.

Insbesondere, wenn ein Zielbereich des Tidalvolumens nicht erreicht wird, d.h. wenn ein gemessenes Tidalvolumen nicht im Solltidalvolumenbereich ist, d.h. kleiner ist als der untere Vt-Sollgrenzwert oder größer ist als der obere Vt-Sollgrenzwert, soll der Solltidalvolumenbereich wieder erreicht werden. Da das Tidalvolumen nicht über die Atemfrequenz aber durch den maximalen Inspirationsdruck angepasst werden kann, kann die Atemfrequenz dem bereits eingestellten Wert entsprechen und lediglich der maximale Inspirationsdruck wird ermittelt. So kann eine Anzahl von Stelleingriffen weiter verringert werden.

Bevorzugt kann das gemessene Minutenvolumen auch durch folgende Gleichung berechnet werden: MV = RR * Vt, wobei MV das ermittelte Minutenvolumen und Vt ein Tidalvolumen ist.

Weiter bevorzugt kann das Tidalvolumen auch durch folgende Gleichung berechnet werden: Vt = (PIP - PEEP) * Crs, wobei PEEP ein positiver endexspiratorischer Druck und Crs die Compliance ist. Ein positiver endexspiratorischer Druck ist der Druck, der am Ende der Exspiration in der Lunge besteht.

In einer bevorzugten Variante der obigen Ausgestaltung hat die Steuereinrichtung mindestens einen vordefinierten Modus hat, der durch die Nutzerschnittstelle auswählbar ist, wobei die Atemgasquellesteuereinheit ausgestaltet ist, die Atemgasquelle basierend auf dem Zielwert des Minutenvolumens unter Verwendung des vordefinierten Modus anzusteuern.

Ein vordefinierter Modus umfasst für die verschiedenen genannten Daten, zum Beispiel einem Zielwert des arteriellen CO2-Partialdrucks, einem ersten vordefinierten Wertebereich, einem zweiten vordefinierten Wertebereich, einem vordefinierten Zeitraum, Patientendaten, einem unteren PIP-Grenzwert, einem oberen PIP-Grenzwert, einem unteren RR-Grenzwert, einem oberen RR-Grenzwert, einem unteren Vt-Sollgrenzwert, einem oberen Vt-Sollgrenzwert etc., vordefinierte Werte und/oder Wertebereiche, so dass diese nicht vom Nutzer eingestellt werden müssen. Dies führt zu einer leichteren Bedienung für den Nutzer, so dass eine potenzielle Anzahl von Fehlern verringert werden kann. Somit kann durch das Verwenden von Modi die Sicherheit des Beatmungsgeräts erhöht werden.

Die obige Variante kann vorteilhafterweise so ausgeführt werden, dass die Atemgasquellesteuereinheit in einem ersten Modus ferner ausgestaltet ist, einen PIP-Wertebereich mit einem unteren PIP-Grenzwert und einem oberen PIP-Grenzwert für den maximalen Inspirationsdruck und einen RR-Wertebereich mit einem unteren RR-Grenzwert und einem oberen RR-Grenzwert für die Atemfrequenz zu erhalten, einen vordefinierten Wert für den maximalen Inspirationsdruck zu erhalten und die Atemgasquelle basierend auf der ermittelten Atemfrequenz anzusteuern, falls der ermittelte Wert für den maximalen Inspirationsdruck kleiner ist als der vordefinierte Wert für den maximalen Inspirationsdruck, wobei die ermittelte Atemfrequenz innerhalb des RR-Wertebereichs liegt.

Dadurch wird ein Modus bereitgestellt, in dem priorisiert eine Atemfrequenz ermittelt und angepasst wird und der maximale Inspirationsdruck unverändert bleibt. Dies ist vor allem dann gewünscht, wenn ein Minutenvolumen erhöht werden soll. D.h. zunächst wird der vordefinierte RR-Wertebereich der Atemfrequenz ausgeschöpft, bevor der maximale Inspirationsdruck ermittelt und angepasst wird. Dies kann zu einer schonenderen Beatmung führen und kann dadurch weiter die Sicherheit des zu beatmenden Patienten verbessern.

Die Variante des ersten Modus kann ferner vorteilhafterweise so ausgeführt sein, dass die Atemgasquellesteuereinheit ausgestaltet ist, die Atemgasquelle basierend auf dem ermittelten maximalen Inspirationsdruck und der ermittelten Atemfrequenz anzusteuern, falls die ermittelte Atemfrequenz einem oberen RR-Grenzwert des RR-Wertebereichs entspricht.

Zum Erreichen des gewünschten Zielwertes des Minutenvolumens wird also solange die Atemfrequenz angepasst, bis ein oberer RR-Grenzwert des RR-Wertebereichs erreicht wird und, wenn der obere RR-Grenzwert des RR-Wertebereichs erreicht ist, der maximale Inspirationsdruck angepasst.

Ferner kann die obige Variante vorteilhafterweise so ausgeführt sein, dass die Atemgasquellesteuereinheit ausgestaltet ist, die Atemgasquelle basierend auf dem ermittelten maximalen Inspirationsdruck und der ermittelten Atemfrequenz anzusteuern, falls der ermittelte Wert für den maximalen Inspirationsdruck größer ist als der vordefinierte Wert für den maximalen Inspirationsdruck.

Insbesondere wird so im ersten Modus in dem Fall, in dem der maximale Inspirationsdruck oberhalb des vordefinierten Werts für den maximalen Inspirationsdruck liegt und das bereitgestellte Minutenvolumen reduziert werden soll, zunächst der maximale Inspirationsdruck reduziert.

Bevorzugt ist die Atemgasquellesteuereinheit ferner ausgestaltet, falls der ermittelte maximale Inspirationsdruck dem vordefinierten Wert für den maximalen Inspirationsdruck entspricht, die Atemgasquelle basierend auf der ermittelten Atemfrequenz anzusteuern, wobei die ermittelte Atemfrequenz innerhalb des RR-Wertebereichs liegt, und, falls die ermittelte Atemfrequenz einem unteren RR-Grenzwert des RR-Wertebereichs entspricht, die Atemgasquelle basierend auf dem ermittelten maximalen Inspirationsdruck anzusteuern. Der erste Modus kann vorzugsweise als ein Basis Modus vorgesehen sein.

Auch dieses Merkmal kann eine schonendere Beatmung für den Patienten in Fällen ermöglichen, in denen zum Beispiel ein Grenzwert eines vorgegebenen Wertebereichs erreicht ist, was wiederum zu einer weiteren Erhöhung der Sicherheit des Patienten führen kann.

Die obige Variante kann vorteilhafterweise so ausgeführt werden, dass die Atemgasquellesteuereinheit in einem zweiten Modus ferner ausgestaltet ist, einen PIP-Wertebereich mit einem unteren PIP-Grenzwert und einem oberen PIP-Grenzwert für den maximalen Inspirationsdruck, einen RR-Wertebereich mit einem unteren RR-Grenzwert und einem oberen RR-Grenzwert für die Atemfrequenz, und ein Solltidalvolumenbereich mit einem unteren Vt-Sollgrenzwert und einem oberen Vt-Sollgrenzwert zu erhalten. Die Atemgasquellesteuereinheit ist vorzugsweise dazu ausgestaltet, basierend auf zumindest dem PIP-Wertebereich, dem RR-Wertebereich und dem Solltidalvolumenbereich einen maximalen Inspirationsdruck zu ermitteln. Die Atemgasquellesteuereinheit steuert vorzugsweise die Atemgasquelle basierend auf dem ermittelten maximalen Inspirationsdruck an, falls ein ermittelter Wert für das Tidalvolumen nicht im Solltidalvolumenbereich liegt. Die Atemgasquellesteuereinheit ist vorzugsweise ferner dazu ausgestaltet, basierend auf zumindest dem PIP-Wertebereich, dem RR-Wertebereich und dem Solltidalvolumenbereich eine Atemfrequenz zu ermitteln. Die Atemgasquellesteuereinheit steuert vorzugsweise die Atemgasquelle basierend auf der ermittelten Atemfrequenz an, falls der ermittelte Wert für das Tidalvolumen innerhalb des Solltidalvolumenbereichs liegt. Der zweite Modus kann somit als Volume-Target Modus bezeichnet werden.

Bevorzugt kann in diesem Modus zusätzlich eine vom Beatmungsgerät gemessene Compliance des Patienten berücksichtigt werden, wobei "gemessen" auch heißt, dass die Compliance von gemessenen Werten berechnet werden kann, zum Beispiel durch Division der Volumenänderung (Tidalvolumen) durch die Druckänderung (PIP - PEEP). Der Nutzer kann hier beispielsweise einen Zielbereich für das Tidalvolumen angeben. Bevorzugt wird ein Zielbereich für das Tidalvolumen abhängig von einem Körpergewicht des Patienten berechnet, wobei in einer Ausführungsform ein Zielbereich von 5 bis 7 ml/kg angegeben wird. In Abhängigkeit von der Compliance des Patienten und des manuell vom ärztlichen Personal gesetzten Positive End-Expiratory Pressure (PEEP) werden ein maximaler Inspirationsdruck und eine Minutenfrequenz ermittelt und basierend auf den ermittelten Werten die Atemgasquelle angesteuert, so dass das vorgegebene Minutenvolumen priorisiert mit dem Zielbereich des Tidalvolumens bereitgestellt wird. Eine Berücksichtigung eines vordefinierten Zielbereichs für das Tidalvolumen kann eine besonders schonende Beatmung ermöglichen und damit die Sicherheit des Patienten weiter erhöhen.

Bevorzugt ist die Atemgasquellesteuereinheit ferner ausgestaltet, die Atemgasquelle basierend auf dem ermittelten maximalen Inspirationsdruck und der ermittelten Atemfrequenz anzusteuern, falls der ermittelte Wert für das Tidalvolumen nicht im Solltidalvolumenbereich liegt und der maximale Inspirationsdruck einem unteren oder oberen PIP-Grenzwert des PIP-Wertebereichs entspricht.

Insbesondere, wenn ein Zielbereich des Tidalvolumens nicht erreicht wird, d.h. wenn ein gemessenes Tidalvolumen nicht im Solltidalvolumenbereich, d.h. kleiner ist als der untere Vt-Sollgrenzwert oder größer ist als der obere Vt-Sollgrenzwert, soll der Solltidalvolumenbereich wieder erreicht werden. Da das Tidalvolumen nicht über die Atemfrequenz aber durch den maximalen Inspirationsdruck angepasst werden kann, kann die Atemfrequenz dem bereits eingestellten Wert entsprechen und lediglich der maximale Inspirationsdruck wird ermittelt. So kann eine Anzahl von Stelleingriffen weiter verringert werden und die Sicherheit des Patienten kann weiter verbessert werden.

Zusätzlich oder alternativ zu dem ersten Modus und dem zweiten Modus können ein oder mehrere weitere Modi vorgesehen sein, bevorzugt ein dritter Modus, wobei die Atemgasquellesteuereinheit bevorzugt ferner ausgestaltet ist, in dem dritten Modus den maximalen Inspirationsdruck und die Atemfrequenz in Abhängigkeit von Patientendaten zu ermitteln, vorzugsweise einer Compliance, einem Widerstand und/oder einem Totraum, so dass eine Atemarbeit eines Patienten minimiert werden kann. Der dritte Modus kann bevorzugt einem Modus entsprechen, wie er aus den folgenden Veröffentlichungen bekannt ist: Otis AB, Fenn WO, Rahn H. Mechanics of breathing in man. J Appl Physiol 1950; 2: 592-607 und Tehrani FT. Method and apparatus for controlling an artificial respirator. US Patent No. 4,986,268, Issued January 22, 1991.

In einem zweiten Aspekt der Erfindung wird eine Steuereinrichtung zur Ansteuerung einer Atemgasquelle eines Beatmungsgeräts vorgeschlagen. Die Steuereinrichtung ist vorzugsweise zur Ansteuerung einer Atemgasquelle eines Beatmungsgeräts vorgesehen, insbesondere für Neugeborene, wobei die Steuereinrichtung vorzugsweise umfasst: eine Zielwertbereitstellungseinheit, die ausgestaltet ist, einen Zielwert des arteriellen CO2-Partialdrucks bereitzustellen, eine Minutenvolumenermittlungseinheit, die ausgestaltet ist, basierend auf dem Zielwert des arteriellen CO2-Partialdrucks und einem ermittelten Wert für den arteriellen CO2-Partialdruck oder einem aus dem endtidalen CO2-Partialdruck abgeleiteten Wert für den arteriellen CO2-Partialdruck einen Zielwert eines Minutenvolumens zu ermitteln, falls der ermittelte Wert für den arteriellen CO2-Partialdruck oder der aus dem endtidalen CO2-Partialdruck abgeleitete Wert für den arteriellen CO2-Partialdruck außerhalb eines ersten vordefinierten Wertebereichs um den Zielwert des arteriellen CO2-Partialdrucks liegt oder über einen Zeitraum kleiner als einem vordefinierten Zeitraum in dem ersten vordefinierten Wertebereich liegt. Die Steuereinrichtung umfasst vorzugsweise eine Atemgasquellesteuereinheit, die ausgestaltet ist, den Zielwert des Minutenvolumens zu empfangen und die Atemgasquelle basierend auf dem Zielwert des Minutenvolumens anzusteuern. In anderen Varianten kann die Steuereinrichtung dazu vorgesehen sein, mit bereits vorhandenen Atemgasquellesteuereinheiten zusammenzuwirken.

In einem dritten Aspekt der Erfindung wir ein Verfahren vorgeschlagen, nämlich ein Verfahren zur Atemgasversorgung, insbesondere von Neugeborenen, umfassend (i) ein Erfassen eines endtidalen CO2-Partialdrucks, (ii) ein Bereitstellen eines Zielwerts des arteriellen CO2-Partialdrucks, (iii) ein Ermitteln, basierend auf dem Zielwert des arteriellen CO2-Partialdrucks und einem ermittelten Wert für den arteriellen CO2-Partialdruck oder einem aus dem endtidalen CO2-Partialdruck abgeleiteten Wert für den arteriellen CO2-Partialdruck, eines Zielwerts eines Minutenvolumens, falls der ermittelte Wert für den arteriellen CO2-Partialdruck oder der aus dem endtidalen CO2-Partialdruck abgeleitete Wert für den arteriellen CO2-Partialdruck außerhalb eines ersten vordefinierten Wertebereichs um den Zielwert des arteriellen CO2-Partialdrucks liegt oder über einen Zeitraum kleiner als einem vordefinierten Zeitraum in dem ersten vordefinierten Wertebereich liegt, (iv) ein Empfangen des Zielwerts des Minutenvolumens und (v) ein Ansteuern der Atemgasquelle basierend auf dem Zielwert des Minutenvolumens anzusteuern.

Nach einem weiteren Aspekt der Erfindung wird ein Computerprogramm mit Programmmitteln vorgeschlagen, die eine Steuerungseinrichtung nach einer der vorstehend beschriebenen Ausführungsformen einer Steuereinrichtung dazu veranlassen, die Schritte des Verfahrens gemäß einer der vorstehend beschriebenen bevorzugten Ausführungsformen eines Verfahrens auszuführen, wenn das Computerprogramm auf der Steuereinrichtung ausgeführt wird.

Das Computerprogramm kann auf einem geeigneten Speichermedium, etwa einem optischen Speichermedium oder einem nicht-flüchtigen elektronischen Speichermedium, vorgesehen, gespeichert und/oder vertrieben werden. Es kann auch zusammen mit oder als Teil einer Hardware-Komponente bereitgestellt werden. Das Computerprogramm kann auch auf andere Weise bereitgestellt werden, etwa über das Internet oder über drahtgebundene oder drahtlose Telekommunikationsmittel.

Merkmale vorteilhafter Ausführungsformen der Erfindung sind insbesondere in den Unteransprüchen definiert, wobei weitere vorteilhafte Merkmale, Ausführungen und Ausgestaltungen für den Fachmann zudem aus den obigen Erläuterung und der folgenden Diskussion zu entnehmen sind.

Im Folgenden wird die vorliegende Erfindung anhand von in den Figuren dargestellten Ausführungsbeispielen weiter illustriert und erläutert.
- Fig. 1: zeigt eine schematische Darstellung zur Illustration eines ersten Ausführungsbeispiels des erfindungsgemäßen Beatmungsgeräts,
- Fig. 2: zeigt ein Blockschaltbild eines Regelkreises, der zur Steuerung des erfindungsgemäßen Beatmungsgeräts verwendet werden kann,
- Fig. 3: zeigt eine schematische Darstellung einer Hierarchie der Steuerungsstruktur, die zur Steuerung des erfindungsgemäßen Beatmungsgeräts verwendet werden kann,
- Fig. 4: zeigt beispielhaft eine Simulation eines Zeitverlaufs eines arteriellen CO2-Partialdrucks und eines Minutenvolumens,
- Fig. 5: zeigt beispielhaft eine Simulation eines Zeitverlaufs eines arteriellen CO2-Partialdrucks, einer Atemfrequenz und eines maximalen Inspirationsdrucks,
- Fig. 6: zeigt eine schematische Darstellung zur Illustration eines weiteren Ausführungsbeispiels des erfindungsgemäßen Beatmungsgeräts,
- Fig. 7: zeigt einen ersten Teil eines schematischen Ablaufdiagramms eines ersten Ausführungsbeispiels des erfindungsgemäßen Verfahrens,
- Fig. 8: zeigt einen zweiten Teil des schematischen Ablaufdiagramms des ersten Ausführungsbeispiels des erfindungsgemäßen Verfahrens,
- Fig. 9: zeigt einen dritten Teil des schematischen Ablaufdiagramms des ersten Ausführungsbeispiels des erfindungsgemäßen Verfahrens,
- Fig. 10: zeigt einen vierten Teil des schematischen Ablaufdiagramms des ersten Ausführungsbeispiels des erfindungsgemäßen Verfahrens, und
- Fig. 11: zeigt ein schematisches Ablaufdiagram eines zweiten Ausführungsbeispiels des erfindungsgemäßen Verfahrens.

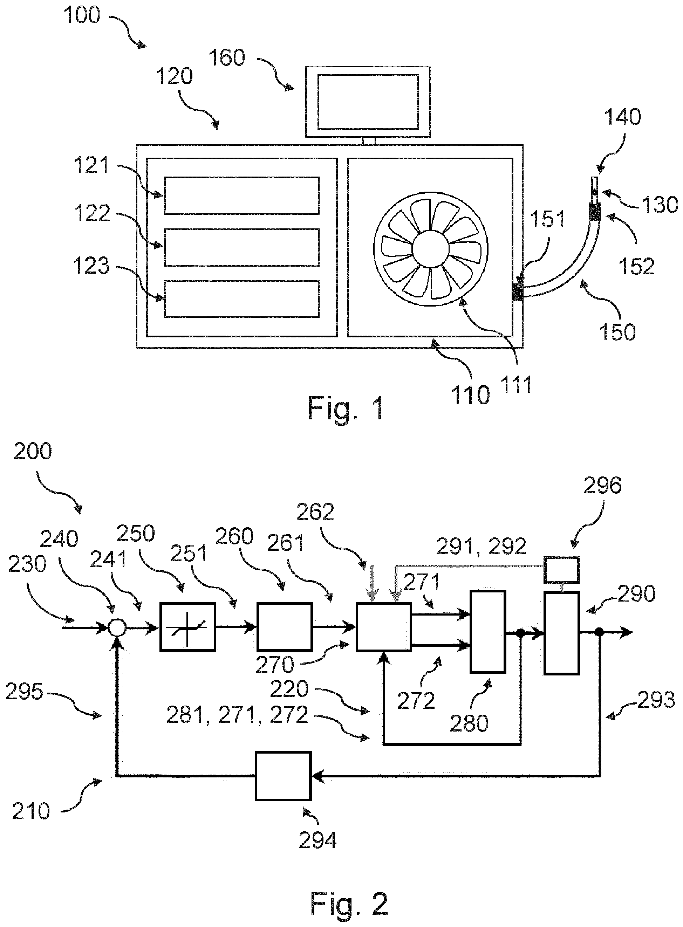

Fig. 1 zeigt eine schematische Darstellung zur Illustration eines ersten Ausführungsbeispiels des erfindungsgemäßen Beatmungsgeräts. Das Beatmungsgerät 100 zur Atemversorgung umfasst eine Atemgasquelle 110, die hier beispielhaft mit einem Ventilator 111 ausgeführt ist, und eine Steuereinrichtung 120 zur Ansteuerung der Atemgasquelle 110. Anstelle des Ventilators 111 können auch andere Atemgas bereitstellende Einheiten verwendet werden. Zudem umfasst das Beatmungsgerät 100 eine Sensoreinrichtung 130, die mit der Steuereinrichtung verbunden ist. Die Sensoreinrichtung 130 ist ausgestaltet, einen endtidalen CO2-Partialdruck, bevorzugt an einem Mundstück 140, zu erfassen. Die Sensoreinrichtung 130 stellt dann Signale, die einen erfassten endtidalen CO2-Partialdruck repräsentieren, an der Steuereinrichtung 120 bereit.

Das Beatmungsgerät 100 kann außerdem einen Atemgasschlauch 150 mit zumindest einem ersten Anschlussstutzen 151 an der Atemgasquelle 110 und einem zweiten Anschlussstutzen 152 für den Atemgasschlauch 150 an dem Mundstück 140 umfassen.

Zudem kann das Beatmungsgerät 100 eine Nutzerschnittstelle 160 umfassen, die ausgestaltet ist, Nutzereingaben, die von einem Nutzer eingegeben werden können, zu empfangen. Des Weiteren ist die Nutzerschnittstelle 160 ausgestaltet, die Nutzereingaben oder von den Nutzereingaben abgeleitete Angaben an die Steuereinrichtung 120 weiterzuleiten.

Die Steuereinrichtung 120 umfasst eine Zielwertbereitstellungseinheit 121, eine Minutenvolumenermittlungseinheit 122 und eine Atemgasquellesteuereinheit 123, die hier konkret als Ventilatoransteuereinheit 123 bezeichnet werden kann. Die Zielwertbereitstellungseinheit 121 ist ausgestaltet, einen Zielwert des arteriellen CO2-Partialdrucks bereitzustellen. Die Zielwertbereitstellungseinheit 121 ist ferner ausgestaltet, den Zielwert des arteriellen CO2-Partialdrucks und der Minutenvolumenermittlungseinheit 122 bereitzustellen. Die Minutenvolumenermittlungseinheit 122 ist ausgestaltet, basierend auf dem Zielwert des arteriellen CO2-Partialdrucks und einem ermittelten Wert für den arteriellen CO2-Partialdruck oder einem aus dem endtidalen CO2-Partialdruck abgeleiteten Wert für den arteriellen CO2-Partialdruck einen Zielwert eines Minutenvolumens zu ermitteln. Die Minutenvolumenermittlungseinheit 122 ist zudem ausgestaltet, ein Minutenvolumen zu ermitteln, insbesondere nur dann zu ermitteln, falls der ermittelte Wert für den arteriellen CO2-Partialdruck oder der aus dem endtidalen CO2-Partialdruck abgeleitete Wert für den arteriellen CO2-Partialdruck außerhalb eines ersten vordefinierten Wertebereichs um den Zielwert des arteriellen CO2-Partialdrucks liegt oder über einen Zeitraum, der kleiner als ein vordefinierter Zeitraum ist, in dem ersten vordefinierten Wertebereich liegt. Die Minutenvolumenermittlungseinheit 122 ist zudem ausgestaltet, den Zielwert für das Minutenvolumen an die Atemgasquellesteuereinheit 123 weiterzuleiten. Die Atemgasquellesteuereinheit 123 ist ausgestaltet, den Zielwert des Minutenvolumens zu empfangen und den Ventilator 111 basierend auf dem Zielwert des Minutenvolumens anzusteuern.

Bevorzugt ist die Atemgasquellesteuereinheit 123 ferner ausgestaltet, einen voreingestellten Wert für das Minutenvolumen zu empfangen und den Ventilator 111 basierend auf dem voreingestellten Wert des Minutenvolumens anzusteuern, falls der ermittelte arterielle CO2-Partialdruck oder der aus dem endtidalen CO2-Partialdruck abgeleitete Wert für den arteriellen CO2-Partialdruck über den vordefinierten Zeitraum in dem ersten vordefinierten Wertebereich liegt, und zudem nach dem vordefinierten Zeitraum in einem zweiten vordefinierten Wertebereich um den Zielwert des arteriellen CO2-Partialdrucks liegt. Dabei ist der der zweite vordefinierte Wertebereich größer als der erste vordefinierte Wertebereich. Vorzugsweise sind der erste und der zweite vordefinierte Wertebereich um denselben Mittelwert angeordnet, bevorzugt dem Zielwert des arteriellen CO2-Partialdrucks.

Weiter bevorzugt ist die Atemgasquellesteuereinheit 123 ausgestaltet, basierend auf dem ermittelten Zielwert des Minutenvolumens einen ersten maximalen Inspirationsdruck und eine erste Atemfrequenz zum Ansteuern des Ventilators 111 zu ermitteln, und den Ventilator 111 basierend auf dem ersten ermittelten maximalen Inspirationsdruck und der ersten ermittelten Atemfrequenz anzusteuern. Weiter ist die Atemgasquellesteuereinheit 123 ausgestaltet, ein gemessenes Minutenvolumen, den ersten ermittelten maximalen Inspirationsdruck und/oder die erste ermittelte Atemfrequenz zu empfangen, basierend zumindest auf dem gemessenen Minutenvolumen, dem ersten ermittelten maximalen Inspirationsdruck und/oder der ersten ermittelten Atemfrequenz einen zweiten maximalen Inspirationsdruck und eine zweite Atemfrequenz zu ermitteln, und den Ventilator 111 basierend auf dem zweiten ermittelten maximalen Inspirationsdruck und der zweiten ermittelten Atemfrequenz anzusteuern. Eine derartige Ansteuerung über einen Regelkreis ist auch in Fig. 2 gezeigt.

Fig. 2 zeigt ein Blockschaltbild 200 eines Regelkreises, der zur Steuerung des erfindungsgemäßen Beatmungsgeräts 100 verwendet werden kann. Der Regelkreis 200 umfasst einen äußeren Regelkreis 210 und einen inneren Regelkreis 220. Eine Führungsgröße 230 umfasst hier einen Zielwert des arteriellen CO2-Partialdrucks. Die Regelung umfasst vorzugsweise einen Vergleich 240 des Zielwerts des arteriellen CO2-Partialdrucks mit einem aus dem endtidalen CO2-Partialdruck abgeleiteten Wert für den arteriellen CO2-Partialdrucks. Eine solche Schätzung beruht vorzugsweise auf einer linearen Näherung aus dem endtidalen CO2-Partialdrucks im Atemgas.

Der aus dem Vergleich 240 resultierende Fehler 241 kann zunächst von einem adaptiven Totzonen-Element 250 gefiltert werden. Dieser im Regelkreis vorgesehene Filter ermöglicht eine Minutenvolumenermittlung wie folgt: Falls die Totzone nicht aktiv ist, d.h. falls der arterielle CO2-Partialdruck außerhalb von 2w liegt oder zwar innerhalb von 2w, liegt aber ein vordefinierter Zeitraum T noch nicht erreicht ist, wird ein Minutenvolumen errechnet auf Basis der Differenz des Zielwerts des arteriellen CO2-Partialdrucks und des aus dem endtidalen CO2-Partialdruck abgeleiteten Wert für den arteriellen CO2-Partialdruck, wobei eine Ableitung insbesondere durch eine Schätzung erfolgt, und an die Atemgasquellesteuereinheit weiter gegeben. Falls die Totzone erreicht ist, d.h. der arterielle CO2-Partialdruck war über einen vordefinierten Zeitraum T innerhalb dem ersten vordefinierten Wertebereich 2w und der arterielle CO2-Partialdruck ist innerhalb des zweiten vordefinierten Wertebereichs 2L, dann wird bevorzugt kein neues Minutenvolumen errechnet sondern das zuvor verwendete Minutenvolumen an die Atemgasquellesteuereinheit weitergegeben.

Der gefilterte Fehler 251 wird an einen Regler 260, bevorzugt einen Pl-Regler, weitergegeben. Der Regler setzt den gefilterten Fehler 251 in einen Zielwert für das Minutenvolumen 261 um. Insbesondere kann das Totzonen-Element 250 und der Regler 260 zusammen als Minutenvolumenermittlungseinheit, zum Beispiel die Minutenvolumenermittlungseinheit 122, verstanden werden. Der Zielwert des Minutenvolumens 261 wird dann an eine Atemgasquellesteuereinheit 270, die zum Beispiel der Atemgasquellesteuereinheit 123 entsprechen und wiederum als Ventilatoransteuereinheit 270 bezeichnet werden kann, weitergeleitet. Die Atemgasquellesteuereinheit 270 ist ausgestaltet, einen ersten maximalen Inspirationsdruck 271 und eine erste Atemfrequenz 272 zu ermitteln und an den Ventilator 280, der zum Beispiel dem Ventilator 111 entsprechen kann, weiterzugeben. Weiter bevorzugt kann die Atemgasquellesteuereinheit 270 ausgestaltet sein, ein gemessenes Minutenvolumen 281, einen ersten ermittelten maximalen Inspirationsdruck 271 und eine erste ermittelte Atemfrequenz 272 zu erhalten und basierend auf zumindest dem gemessenen Minutenvolumen 281, dem ersten ermittelten maximalen Inspirationsdruck 271 und der ersten ermittelten Atemfrequenz 272 einen zweiten maximalen Inspirationsdruck und eine zweite Atemfrequenz zu ermitteln. Bevorzugt kann der zweite maximale Inspirationsdruck bzw. die zweite Atemfrequenz als jeder weitere ermittelte maximale Inspirationsdruck bzw. jede weitere ermittelte Atemfrequenz verstanden werden. In einer weiteren Variante kann die Ventilationssteuereinheit auch ausgestaltet sein, zur Ermittlung eines maximalen Inspirationsdrucks und einer Atemfrequenz einen Totraum 262 zu berücksichtigen, der den Raum des Atemsystems repräsentiert, der nicht am pulmonalen Gasaustausch beteiligt ist. Insbesondere können auch weitere Parameter eines Patienten 296 genutzt werden, zum Beispiel eine Compliance 291 und/oder ein Widerstand 292 des Patienten 296.

Der Ventilator 280 nutzt die Werte des ersten maximalen Inspirationsdrucks und der ersten Atemfrequenz bzw. des zweiten maximalen Inspirationsdrucks und der zweiten Atemfrequenz zur Verwendung des Beatmungsgeräts, d.h. zur Bereitstellung von Atemluft durch das Mundstück 290 für einen Patienten 296. Zudem wird vorzugsweise ein endtidaler CO2-Partialdruck 293 bei Verwenden des Beatmungsgeräts 100 an dem Mundstück 290 durch eine Sensoreinrichtung erfasst und zur Abschätzung 294 des arteriellen CO2-Partialdrucks 295 verwendet. Somit wird eine closed-loop Beatmung mit einer automatischen Regelung des arteriellen CO2-Partialdrucks bereitgestellt.

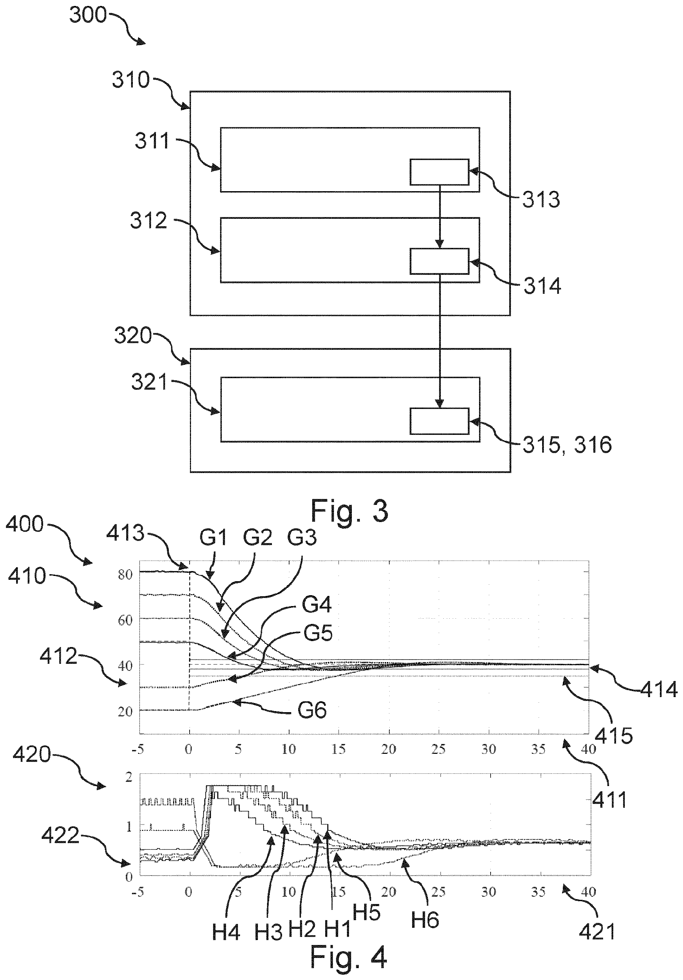

Fig. 3 zeigt eine schematische Darstellung einer Hierarchie der Steuerungsstruktur, die zur Steuerung des erfindungsgemäßen Beatmungsgeräts 100 verwendet werden kann. Insbesondere umfasst die Steuerungsstruktur 300 eine kaskadierte Steuerung 310 und einen Ventilator 320, der beispielsweise dem Ventilator 111 entspricht. Die kaskadierte Steuerung 31 umfasst einen äußeren Regelkreis 311 und einen inneren Regelkreis 312. Der äußere Regelkreis 311 stellt den übergeordneten Regler dar, der einen angemessenen Gasaustausch aufrechterhält. Der innere Regelkreis 312 stellt den mittleren Regler dar und zielt auf eine sichere Beatmung ab. Eine untere Steuerung 321 umfasst die konkrete Ansteuerung des Ventilators zur Durchführung der Beatmung, d.h. beispielsweise eine Steuerung der Belüftung in Bezug auf Druck, Durchfluss und Volumen des Beatmungsgases. Erfindungsgemäß wird ein arterieller CO2-Partialdruck 313 im äußeren Regelkreis in ein Minutenvolumen 314 im inneren Regelkreis 312 umgesetzt, das wiederum in einen maximalen Inspirationsdruck 315 und eine Atemfrequenz 316 umgesetzt wird.

Fig. 4 zeigt beispielhaft eine Simulation 400 eines Zeitverlaufs eines arteriellen CO2-Partialdrucks und eines Minutenvolumens. Insbesondere zeigt Fig. 4 ein Diagramm 410, bei dem auf der horizontalen Achse 411 eine Zeit in Minuten und auf der vertikalen Achse 412 ein arterieller CO2-Partialdruck in mmHg angegeben ist. Des Weiteren zeigt Fig. 4 ein Diagramm 420, bei dem auf der horizontalen Achse 421 eine Zeit in Minuten und auf der vertikalen Achse 422 ein Minutenvolumen in Litern angegeben ist. Die Diagramme zeigen simulierte Sprungantworten auf verschiedenen Ebenen einer Hyperkapnie bzw. Hypokapnie, d.h. eines erhöhten bzw. herabgesetzten CO2-Partialdrucks. Jede Linie G1, G2, G3, G4, G5, G6 (im Diagramm 410 im linken Bereich von oben nach unten) repräsentiert den Verlauf des arteriellen Partialdrucks in einem Experiment und jede Linien H1, H2, H3, H4 H5, H6 (im Diagramm 420 zugehörig zu der jeweiligen Linie G1 bis G6) ein entsprechendes Minutenvolumen. Bei t = 0 min ist ein Sprung des Referenzsignals vom jeweiligen stationären Wert auf 45 mmHg appliziert, markiert durch die vertikale gestrichelte Linie 413. Der schattierte Bereich 414, der durch kleine Punkte markiert ist und in dem die verschiedenen Linien G1 bis G6 in diesem Beispiel spätestens nach 20 Minuten gelangt sind, markiert die adaptive Totzone, d.h. die Zielzone. Die dick gepunktete Linie 415 steht für eine imaginäre 35 mmHg-Schwelle, die nicht aufgrund von Reglereingriffen, z. B. beim Unterschreiten, überfahren werden darf. Insbesondere zeigt der Verlauf des arteriellen CO2-Partialdrucks nach Eingeben eines geänderten Zielwerts des arteriellen Partialdrucks ein Einpendeln des arteriellen CO2-Partialdrucks in der Totzone. Im Allgemeinen ist ein Einpendeln bei einer Hyperkapnie (G1-G4) schneller als bei einer Hypokapnie (G5, G6) aufgrund der unteren Grenze der manipulierten Variablen, die schneller und länger erreicht werden.

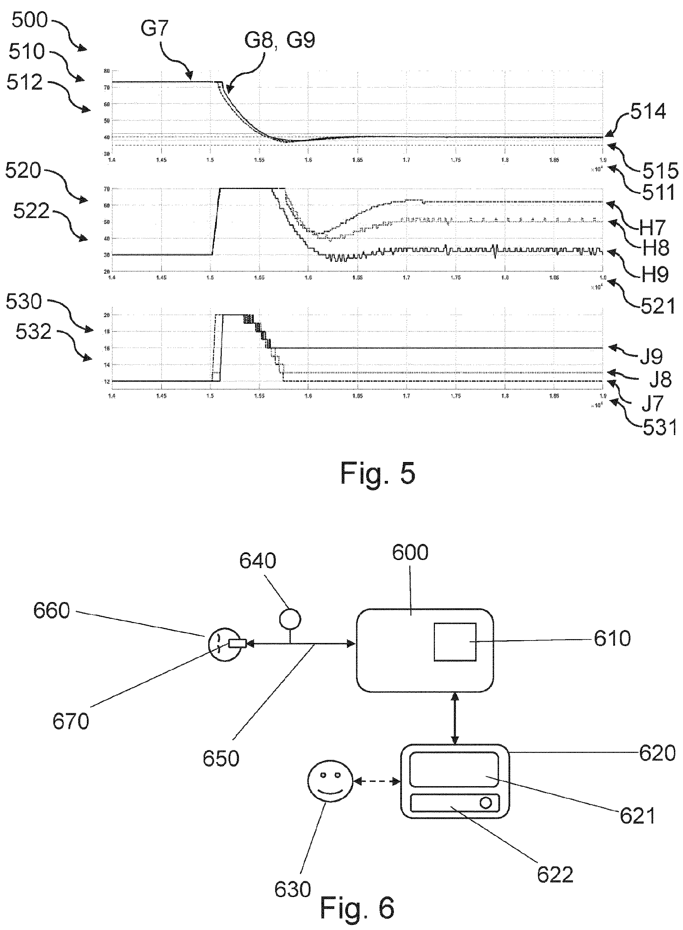

Fig. 5 zeigt beispielhaft eine Simulation 500 eines Zeitverlaufs eines arteriellen CO2-Partialdrucks, einer Atemfrequenz und eines maximalen Inspirationsdrucks. Insbesondere zeigt Fig. 5 ein Diagramm 510, bei dem auf der horizontalen Achse 511 eine Zeit in Sekunden angegeben ist und auf der vertikalen Achse 512 ein arterieller CO2-Partialdruck in mmHg. Des Weiteren zeigt Fig. 5 ein Diagramm 520, bei dem auf der horizontalen Achse 521 eine Zeit in Sekunden angegeben ist und auf der vertikalen Achse 522 eine Atemfrequenz RR pro Minute. Zudem zeigt Fig. 5 ein Diagramm 530, bei dem auf der horizontalen Achse 531 eine Zeit in Sekunden angegeben ist und auf der vertikalen Achse 532 ein maximaler Inspirationsdruck in mbar.

In dem Diagramm 510 ist wiederum ein Totzeitbereich als schraffierter Bereich mit kleinen Punkten eingezeichnet, der auch als Zielbereich für den arteriellen CO2-Partialdruck verstanden werden kann. Des Weiteren ist im Diagramm 510 eine untere Grenze als lang gestrichelte Linie 515 dargestellt. In den Diagrammen 510, 520 und 530 werden zwei Modi des Beatmungsgeräts gezeigt, wobei der erste Modus durch die Graphen G7, H7, J7, der zweite Modus durch die Graphen G8, H8, J8, und der dritte Modus durch die Graphen G9, H9, J9 gekennzeichnet ist. Der Zeitverlauf des arteriellen CO2-Partialdrucks im dritten Modus entspricht unter Berücksichtigung der Genauigkeit des Diagramms etwa dem Zeitverlauf des arteriellen CO2-Partialdrucks im zweiten Modus, so dass der Zeitverlauf des arteriellen CO2-Partialdrucks im dritten Modus, der vor allem als Referenz dient, im Diagramm 510 nicht zu sehen ist.

Zuerst ist das Modell durch Beatmung in eine stationäre Hyperkapnie von 73 mmHg überführt worden durch Beatmung mit einem maximalen Inspirationsdruck von 12 mmHg und einer Atemfrequenz von 30 pro Minute für 15 000 s (in der Figur nicht gezeigt). In diesem Zustand wird der Regler bei t=15 000 s mit moderater Parametrisierung aktiviert. Die Reaktionszeit ist bei allen drei Modi ähnlich, wobei die Reaktionszeit im ersten Modus mit 505 s schneller ist als die Reaktionszeit mit 545 s im zweiten bzw. dritten Modus. Insbesondere liegt eine schnellere Reaktionszeit des ersten Modus daran, dass beide Ausgabeparameter, d.h. der maximale Inspirationsdruck und die Atemfrequenz, im ersten Modus verändert werden können, während im zweiten Modus und im dritten Modus jeweils nur ein Parameter verändert wird. Alle drei Modi führen zu Unterschreitungen zwischen 36,5 und 37,5 mmHg und verlaufen im Zielbereich. Die Beatmungsparameter, d.h. der maximale Inspirationsdruck und die Atemfrequenz, bei denen der Zielbereich erreicht wird, unterscheiden sich jedoch deutlich, wie aus den Diagrammen 520 und 530 zu erkennen.

Nachdem der maximale Inspirationsdruck und die Atemfrequenz auf das Maximum gestellt wurden, d.h. dem oberen PIP-Grenzwert und dem oberen RR-Grenzwert entsprechen, um den arteriellen CO2-Partialdruck in allen Modi zu verringern, treten unterschiedliche Kombinationen des maximalen Inspirationsdrucks und der Atemfrequenz auf. Beispielsweise zeigt der erste Modus (G7, H7, J7) eine Kombination aus höchster Atemfrequenz und minimal möglichem maximalen Inspirationsdruck. Die Referenz, d.h. im dritten Modus (G9, H9, J9), pendelt sich dagegen bei einem deutlich höheren maximalen Inspirationsdruck und einer niedrigeren Atemfrequenz ein. Dazwischen liegt der zweite Modus. Die Ausgangswerte des dritten und zweiten Modus entsprechen ihren Einstellungen. Für den zweiten Modus umfassen die Einstellungen eine Auswahl des maximalen Inspirationsdrucks entsprechend vordefinierter Compliance- und Volumenzielen. Für die Referenz, d.h. den dritten Modus, bedeutet dies, den maximalen Inspirationsdruck nicht unter eine Priorisierungsschwelle von 16 mbar zu senken, wenn möglich. Welche Parameter sich letztendlich in diesen beiden Modi ergeben, hängt also von den Einstellungen ab, die Nutzer voreingestellt hat.

Fig. 6 zeigt eine schematische Darstellung zur Illustration eines weiteren Ausführungsbeispiels des erfindungsgemäßen Beatmungsgeräts. In Fig. 6 ist das Beatmungsgerät 600 mit einer Steuerungseinrichtung 610 ausgestattet, das in dem Beatmungsgerät 600 integriert ist. Die Steuerungseinrichtung 610 kann aber alternativ auch außerhalb des Beatmungsgeräts 600 vorgesehen sein und über eine Kommunikation mit Kabel oder kabellos mit dem Beatmungsgerät 600 verbunden sein. Das Beatmungsgerät 600 umfasst ein Nutzerinterface 620. Das Nutzerinterface 620 kann als Teil des Beatmungsgeräts 600 oder separat von dem Beatmungsgerät 600 vorgesehen sein. Das Nutzerinterface 620 umfasst in diesem Ausführungsbeispiel einen Display 621 und eine Eingabeeinrichtung 622, das zum Beispiel ein Touchscreen, Knöpfe, eine Maus und/oder eine Tastatur umfassen kann. Über die Eingabeeinrichtung 622 kann ein Nutzer 630 Daten eingeben und bevorzugt die Regler und das Beatmungsgerät 600 über das Nutzerinterface 620 einstellen und/oder überwachen. Bevorzugt umfasst das Beatmungsgerät 600 einen Sensor bzw. eine Sensoreinrichtung 640 zur Erfassung eines endtidalen CO2-Partialdrucks, der bzw. die in einem Beatmungskreislauf 650 vorgesehen ist. Der Sensor bzw. die Sensoreinrichtung 640 kann dabei in einem Hauptstrom oder einem Nebenstrom der Atemluft vorgesehen sein. Ein Patient 660 kann mit dem Beatmungsgerät 600 über ein Mundstück oder einen Tubus 670 mit dem erfindungsgemäßen Beatmungsgerät verbunden sein. Die Steuereinrichtung 610 ist dabei ausgestaltet, einen Zielwert für einen arteriellen CO2-Partialdruck bereitzustellen. Die Steuereinrichtung ist ferner ausgestaltet, basierend auf dem Zielwert des arteriellen CO2-Partialdrucks und einem ermittelten Wert für den arteriellen CO2-Partialdruck oder einem aus dem endtidalen CO2-Partialdruck abgeleiteten Wert für den arteriellen CO2-Partialdruck einen Zielwert eines Minutenvolumens zu ermitteln, falls der ermittelte Wert für den arteriellen CO2-Partialdruck oder der aus dem endtidalen CO2-Partialdruck abgeleitete Wert für den arteriellen CO2-Partialdruck außerhalb eines ersten vordefinierten Wertebereichs um den Zielwert des arteriellen CO2-Partialdrucks liegt oder über einen Zeitraum kleiner als einem vordefinierten Zeitraum in dem ersten vordefinierten Wertebereich liegt. Ferner ist die Steuerungseinrichtung 610 ausgestaltet, den Zielwert des Minutenvolumens zu empfangen und einen Ventilator basierend auf dem Zielwert des Minutenvolumens anzusteuern.

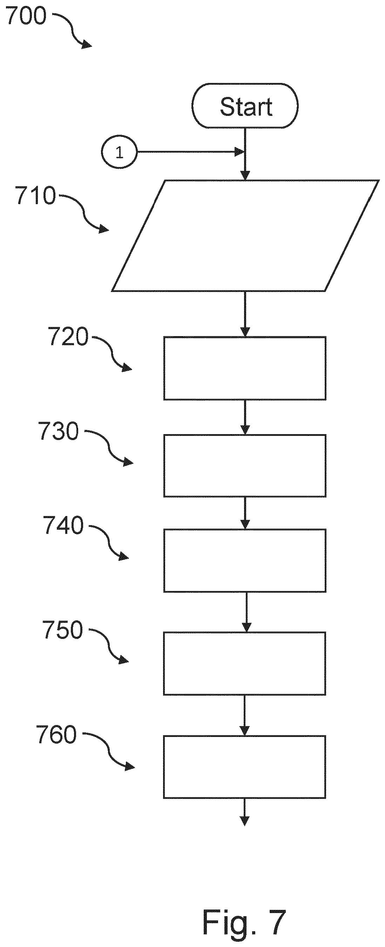

Fig. 7 zeigt einen ersten Teil eines schematischen Ablaufdiagramms eines ersten Ausführungsbeispiels des erfindungsgemäßen Verfahrens. Insbesondere kann das Verfahren 700 in einem ersten Schritt 710 ein Einlesen von Nutzeingaben mit einem Zielwert für einen arteriellen CO2-Partialdrucks PaCO2-Ziel umfassen und vorzugsweise Werte für die Parameter C1 und C2. Zusätzlich kann vorzugsweise ein Messwert für einen endtidalen CO2-Partialdruck und ein Minutenvolumen eingelesen werden. In einem zweiten Schritt 720 kann dann vorzugsweise ein aus dem endtidalen CO2-Partialdruck abgeleiteter Wert Pa^CO2 berechnet werden. In einem dritten Schritt 730 kann dann ein Fehler e(PaCO2)=PaCO2-Ziel - Pa^CO2 berechnet werden. In einem vierten Schritt 740 wird dann ein gefilterter Fehler e^PaCO2 berechnet, wie oben bereits beschrieben worden ist. In einem fünften Schritt 750 wird dann ein Zielwert des Minutenvolumens MVZiel berechnet, wobei dann in einem sechsten Schritt bevorzugt ein Fehler des Minutenvolumens eMV=MVZiel - MV berechnet werden kann.

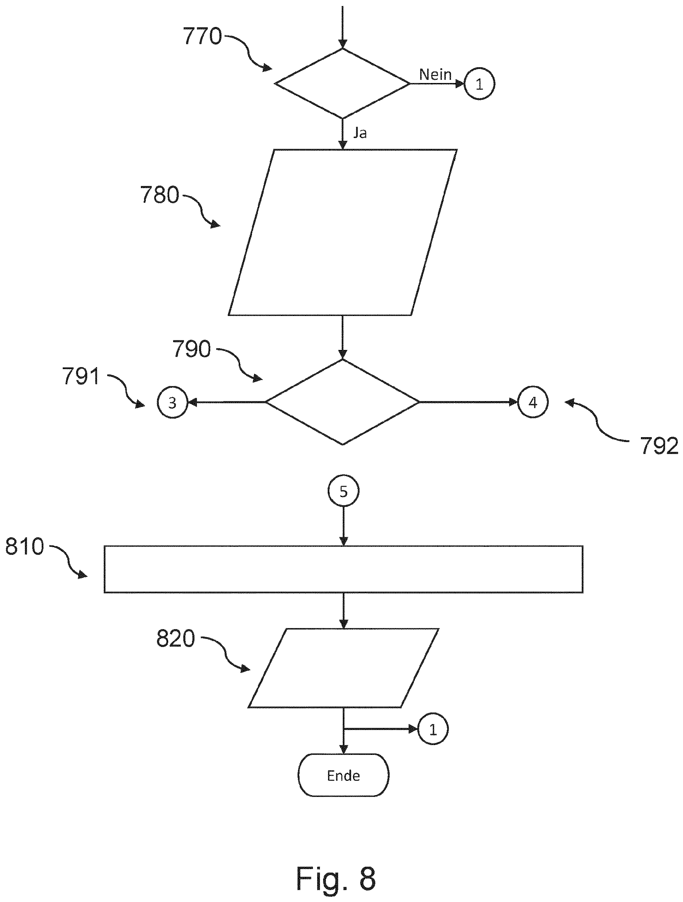

Fig. 8 zeigt einen zweiten Teil des schematischen Ablaufdiagramms des ersten Ausführungsbeispiels des erfindungsgemäßen Verfahrens. Vorzugsweise wird in einem nächsten Schritt 770, der in Fig. 8 gezeigt ist, ein Betrag des Fehlers des Minutenvolumens | eMV| berechnet. Wenn der Betrag des Fehlers des Minutenvolumens nicht größer als null ist, wird, beginnt das erfindungsgemäße Verfahren von vorne mit den oben genannten Schritten. Ist ein Betrag des Fehlers des Minutenvolumens größer als null, d.h. | eMV| > 0, so geht das Verfahren über zu einem nächsten Schritt 780. Im Schritt 780 werden Nutzerdaten eingelesen, zum Beispiel ein Minutenvolumen-Regler-Modus, ein unterer RR-Grenzwert und ein oberer RR-Grenzwert für eine Atemfrequenz, und/oder ein unterer PIP-Grenzwert und ein oberer PIP-Grenzwert für einen maximalen Inspirationsdruck. Zudem werden im Schritt 780 bevorzugt aktuelle Einstellungen des Beatmungsgeräts wie die aktuelle Atemfrequenz, der aktuelle maximale Inspirationsdruck sowie der PEEP eingelesen. In einem nächsten Schritt 790 kann dann ein Modus ausgewählt werden, insbesondere ein erster Modus 791, der als ein Basis-Modus verstanden werden kann oder ein zweiter Modus 792, der als ein Volume-Target-Modus verstanden werden kann.

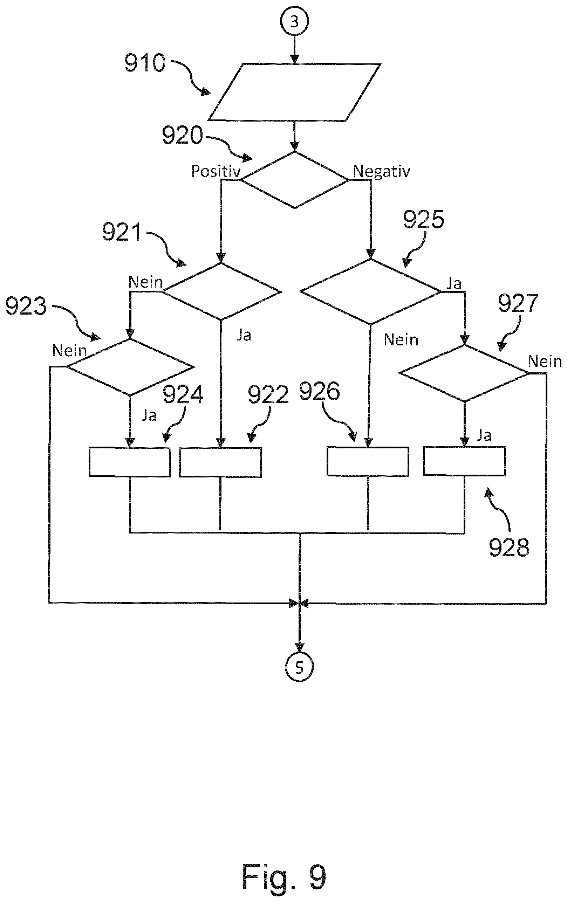

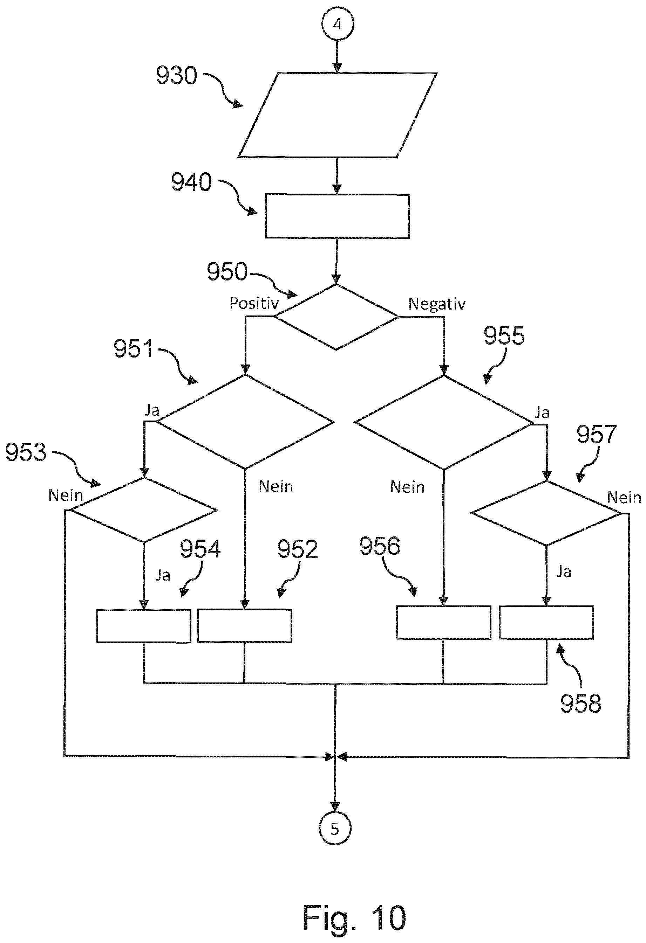

Durch den jeweiligen Modus, die weiter unten im Zusammenhang mit Fig. 9 und 10 genauer beschrieben werden, kann dann ein neu berechneter Zielwert für die Atemfrequenz und den maximalen Inspirationsdruck in einem Schritt 810 auf Überschreitung der Sicherheitsgrenzen, d.h. auf Überschreiten des unterer RR-Grenzwerts und/oder eines oberen RR-Grenzwerts für die Atemfrequenz, und/oder des unteren PIP-Grenzwerts und des oberen PIP-Grenzwerts für den maximalen Inspirationsdruck überprüft werden. Bevorzugt werden in Schritt 810 die neu berechneten Zielwerte angepasst, wenn der untere RR-Grenzwert bzw. der untere PIP-Grenzwert unterschritten wird und/oder der obere RR-Grenzwert bzw. der obere PIP-Grenzwert überschritten wird. In einem Schritt 820 werden dann neue Werte für den maximalen Inspirationsdruck und die Atemfrequenz geschrieben und bevorzugt an die Atemgasquellesteuereinheit zur Ansteuerung des Ventilators ausgegeben. Nach Schreiben der neuen Werte für den maximalen Inspirationsdruck und die Atemfrequenz kann das Verfahren 700 dann wieder von vorne beginnen, d.h. mit Schritt 710.

Bevorzugt kann ein Anpassen der Atemfrequenz und des maximalen Inspirationsdrucks in festgelegter Schrittweite erfolgen oder jeweils anhand eines geeigneten Reglers, z.B. einem diskreten Pl-Regler, welcher einen von einem Nutzer eingestellten Schrittweitenbereich nutzt.

Fig. 9 zeigt einen dritten Teil des schematischen Ablaufdiagramms des ersten Ausführungsbeispiels des erfindungsgemäßen Verfahrens. Fig. 9 zeigt insbesondere das Verfahren für den oben beschrieben ersten Modus, d.h. den Basis-Modus. In einem ersten Schritt 910 im ersten Modus werden Nutzereingaben, die mindestens einen vordefinierten Wert für den maximalen Inspirationsdruck PIPprio umfassen, eingelesen. In einem Schritt 920 wird geprüft, ob ein Fehler des Minutenvolumens eMV positiv oder negativ ist. Ist ein Fehler des Minutenvolumens eMV positiv, so wird in einem Schritt 921 geprüft, ob eine Atemfrequenz RR einem oberen RR-Grenzwert RRmax entspricht. Ist die Atemfrequenz RR kleiner als der obere RR-Grenzwert RRmax, d.h. RR < RRmax, so wird die Atemfrequenz RR in einem Schritt 922 erhöht und als nächster Schritt der Schritt 820 ausgeführt, d.h. neue Werte für den maximalen Inspirationsdruck und die Atemfrequenz geschrieben und zur Ansteuerung des Ventilators verwendet. Ist die Atemfrequenz RR nicht kleiner als der obere RR-Grenzwert RRmax, so wird in einem Schritt 923 geprüft, ob der maximale Inspirationsdruck PIP kleiner als ein oberer PIP-Grenzwert PIPmax ist. Ist der maximale Inspirationsdruck PIP kleiner als der obere PIP-Grenzwert, d.h. PIP < PIPmax, so wird der maximale Inspirationsdruck in einem Schritt 924 erhöht und im Anschluss Schritt 820 ausgeführt, d.h. neue Werte für den maximalen Inspirationsdruck und die Atemfrequenz geschrieben und zur Ansteuerung des Ventilators verwendet. Ist der maximale Inspirationsdruck PIP nicht kleiner als der obere PIP-Grenzwert, so wird in der Folge Schritt 820, der bereits oben beschrieben wurde, ausgeführt und bevorzugt neue Werte für den maximalen Inspirationsdruck und die Atemfrequenz geschrieben und zur Ansteuerung des Ventilators verwendet.

Ist ein Fehler des Minutenvolumens eMV negativ, so wird in einem Schritt 925 geprüft, ob ein maximaler Inspirationsdruck PIP größer ist als der vordefinierte Wert für den maximalen Inspirationsdruck PIPprio, d.h. PIP > PIPprio oder ob die Atemfrequenz RR dem unteren RR-Grenzwert entspricht, RR == RRmin. Ist keine der genannten Bedingungen erfüllt, wird die Atemfrequenz RR in einem Schritt 926 gesenkt und als nächster Schritt der Schritt 820 ausgeführt, d.h. neue Werte für den maximalen Inspirationsdruck und die Atemfrequenz geschrieben und zur Ansteuerung des Ventilators verwendet. Ist eine der oben genannten Bedingungen erfüllt, so wird in einem Schritt 927 geprüft, ob der maximale Inspirationsdruck PIP größer ist als der untere PIP-Grenzwert PIPmin, d.h. ob PIP > PIPmin gilt. Ist der maximale Inspirationsdruck PIP größer als der untere PIP-Grenzwert PIPmin, so wird der maximale Inspirationsdruck in einem Schritt 928 gesenkt und als nächster Schritt der Schritt 820 ausgeführt, d.h. neue Werte für den maximalen Inspirationsdruck und die Atemfrequenz geschrieben und zur Ansteuerung des Ventilators verwendet. Ist der maximale Inspirationsdruck PIP nicht größer als der untere PIP-Grenzwert PIPmin, so wird als nächster Schritt der Schritt 820 ausgeführt, d.h. neue Werte für den maximalen Inspirationsdruck und die Atemfrequenz geschrieben und zur Ansteuerung des Ventilators verwendet.

Fig. 10 zeigt einen vierten Teil des schematischen Ablaufdiagramms des ersten Ausführungsbeispiels des erfindungsgemäßen Verfahrens. Fig. 10 zeigt insbesondere das Verfahren für den oben beschrieben zweiten Modus, d.h. den Volume-Target-Modus. In einem ersten Schritt 930 im zweiten Modus werden Nutzereingaben eingelesen, insbesondere ein bevorzugter unterer Sollgrenzwert für das Tidalvolumen Vtpl und ein bevorzugter oberer Sollgrenzwert für das Tidalvolumen Vtpu. Bevorzugt wird zudem in Schritt 930 ein Messwert der Compliance Crs des Patienten eingelesen. In einem nächsten Schritt 940 wird ein Zielwert für ein Tidalvolumen Vt eingelesen oder berechnet. In einem Schritt 950 wird geprüft, ob ein Fehler des Minutenvolumens eMV positiv oder negativ ist. Ist ein Fehler des Minutenvolumens eMV positiv, so wird in einem Schritt 951 geprüft, ob das Tidalvolumen Vt kleiner als der bevorzugte untere Sollgrenzwert Vtpl ist, und ob der maximale Inspirationsdruck PIP kleiner als der obere PIP-Grenzwert ist, d.h., ob Vt < Vtpl und PIP < PIPmax gilt oder ob eine Atemfrequenz RR gleich einem oberen RR-Grenzwert ist, d.h. ob RR == RRmax gilt. Ist keine der genannten Bedingungen erfüllt, so wird in einem Schritt 952 die Atemfrequenz erhöht und in der Folge Schritt 820 ausgeführt, d.h. neue Werte für den maximalen Inspirationsdruck und die Atemfrequenz geschrieben und zur Ansteuerung des Ventilators verwendet. Ist eine der genannten Bedingungen erfüllt, d.h. gilt (Vt < Vtpl und PIP < PIPmax) oder RR == RRmax, so wird in einem Schritt 953 geprüft, ob ein maximaler Inspirationsdruck PIP kleiner als ein oberer PIP-Grenzwert PIPmax ist, d.h., ob PIP < PIPmax gilt. Ist der maximale Inspirationsdruck PIP kleiner als der obere PIP-Grenzwert PIPmax, so wird der maximale Inspirationsdruck PIP in einem Schritt 954 erhöht und anschließend Schritt 820 ausgeführt, d.h. neue Werte für den maximalen Inspirationsdruck und die Atemfrequenz geschrieben und zur Ansteuerung des Ventilators verwendet. Ist der maximale Inspirationsdruck PIP nicht kleiner als der obere PIP-Grenzwert PIPmax, so wird als nächster Schritt der Schritt 820 ausgeführt, d.h. neue Werte für den maximalen Inspirationsdruck und die Atemfrequenz geschrieben und zur Ansteuerung des Ventilators verwendet. Ist ein Fehler des Minutenvolumens eMV beim Prüfen im Schritt 950 negativ, so wird in einem Schritt 955 geprüft, ob das Tidalvolumen Vt größer ist als ein bevorzugter oberer Sollgrenzwert für das Tidalvolumen Vtpu und ob ein maximaler Inspirationsdruck PIP größer ist als ein unterer PIP-Grenzwert PIPmin, d.h. ob Vt > Vtpu und PIP > PIPmin gilt, oder ob eine Atemfrequenz gleich einem unterem RR-Grenzwert ist, d.h. ob RR == RRmin gilt. Ist keine der genannten Bedingungen erfüllt, so wird in einem Schritt 956 die Atemfrequenz gesenkt und anschließend Schritt 820 ausgeführt, d.h. neue Werte für den maximalen Inspirationsdruck und die Atemfrequenz geschrieben und zur Ansteuerung des Ventilators verwendet. Ist eine der genannten Bedingungen erfüllt, d.h. gilt (Vt > Vtpu und PIP > PIPmin) oder RR == RRmin, so wird in einem Schritt 957 geprüft, ob der maximale Inspirationsdruck PIP größer als ein unterer PIP-Grenzwert PIPmin ist, d.h., ob PIP > PIPmin gilt. Ist der maximale Inspirationsdruck PIP größer als der untere PIP-Grenzwert PIPmin, so wird in einem Schritt 958 der maximale Inspirationsdruck gesenkt und als nächster Schritt der Schritt 820 ausgeführt, d.h. neue Werte für den maximalen Inspirationsdruck und die Atemfrequenz geschrieben und zur Ansteuerung des Ventilators verwendet. Ist der maximale Inspirationsdruck PIP nicht größer als der untere PIP-Grenzwert PIPmin, so wird als nächster Schritt der Schritt 820 ausgeführt, d.h. neue Werte für den maximalen Inspirationsdruck und die Atemfrequenz geschrieben und zur Ansteuerung des Ventilators verwendet.

Es ist anzumerken, dass es sich bei den bevorzugten unteren und oberen Sollgrenzwerten Vtpl und Vtpu, im Gegensatz zu den Grenzwerten PIPmin, PIPmax, RRmin und RRmax, nicht um harte Grenzen handelt, sondern um Grenzen eines Zielbereichs, deren Einhaltung angestrebt wird, die im Fall der Notwendigkeit stärkerer Beatmung aber auch überschritten werden können.

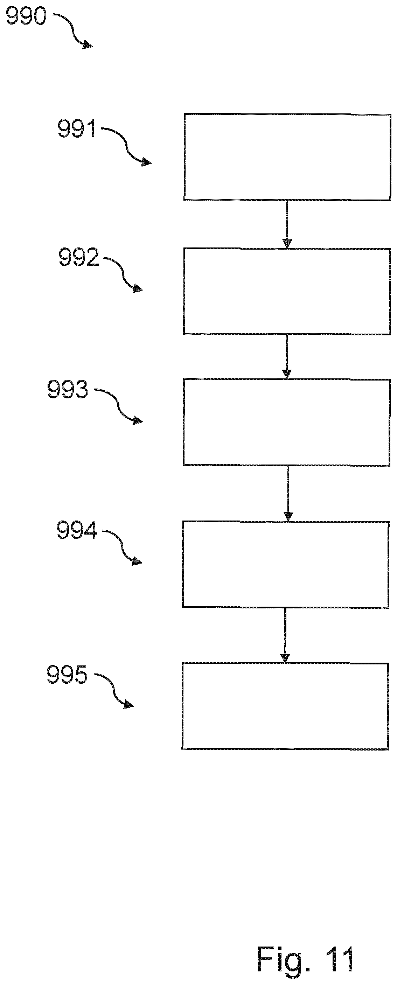

Fig. 11 zeigt ein schematisches Ablaufdiagramm eines zweiten Ausführungsbeispiels des erfindungsgemäßen Verfahrens. Insbesondere ist das erfindungsgemäße Verfahren ein Verfahren 990 zur Atemgasversorgung, insbesondere von Neugeborenen, umfassend einen ersten Schritt 991 eines Erfassens eines endtidalen CO2-Partialdrucks. Zudem umfasst das Verfahren 990 einen zweiten Schritt 992 eines Bereitstellens eines Zielwerts des arteriellen CO2-Partialdrucks. In dritter Schritt 993 des Verfahrens 990 umfasst dann ein Ermitteln eines Zielwerts eines Minutenvolumens, basierend auf dem Zielwert des arteriellen CO2-Partialdrucks und einem ermittelten Wert für den arteriellen CO2-Partialdruck oder einem aus dem endtidalen CO2-Partialdruck abgeleiteten Wert für den arteriellen CO2-Partialdruck, falls der ermittelte Wert für den arteriellen CO2-Partialdruck oder der aus dem endtidalen CO2-Partialdruck abgeleitete Wert für den arteriellen CO2-Partialdruck außerhalb eines ersten vordefinierten Wertebereichs um den Zielwert des arteriellen CO2-Partialdrucks liegt oder über einen Zeitraum kleiner als einem vordefinierten Zeitraum in dem ersten vordefinierten Wertebereich liegt. Bevorzugt umfasst das Verfahren 990 in einem Schritt 994 ein Empfangen des Zielwerts des Minutenvolumens und in einem Schritt 995 ein Ansteuern des Ventilators basierend auf dem Zielwert des Minutenvolumens.

Auch wenn in den Figuren verschiedene Aspekte oder Merkmale der Erfindung jeweils in Kombination gezeigt sind, ist für den Fachmann - soweit nicht anders angegeben - ersichtlich, dass die dargestellten und diskutieren Kombinationen nicht die einzig möglichen sind. Insbesondere können einander entsprechende Einheiten oder Merkmalskomplexe aus unterschiedlichen Ausführungsbeispielen miteinander ausgetauscht werden.

In Implementierungen der Erfindung können jeweils einzelne Komponenten, z.B. ein Prozessor, ganz oder teilweise die Funktionen verschiedener in den Ansprüchen genannter Elemente übernehmen. Abläufe oder Vorgänge können als Programmmittel eines Computerprogramms und/oder als spezielle Hardwarekomponenten implementiert werden.

Es folgen weitere Überlegungen zu Aspekten der Erfindung.

Die Erfindung umfasst eine algorithmische Methode und deren Implementierung in Software zur closed-loop Beatmung, bevorzugt von Neugeborenen, mit dem Ziel der automatischen Regelung des arteriellen CO2-Partialdrucks PaCO2 bei gleichzeitiger Schonung der Lunge bei der künstlichen Beatmung, bevorzugt Neugeborener. Der Algorithmus nutzt bevorzugt eine kaskadierte Regelkreisstruktur, wie in Fig. 2 gezeigt, wobei die äußere Schleife der Regelung des PaCO2 durch das Minutenvolumen MV dient, während in der inneren Schleife das MV durch die Beatmungsparameter Respiratory Rate RR, d.h. Atemfrequenz, und Peak Inspiratory Pressure PIP, d.h. maximaler Inspirationsdruck, eingestellt wird. Die Ausführungsfrequenz liegt bevorzugt für den äußeren Regelkreis bei 1/20 Hz und für den inneren Regelkreis bei 1/4 Hz.

Bevorzugt wird für die Erfindung eine kaskadierter Regelkreis vorgesehen: Zunächst wird bevorzugt ein Minutenvolumen in einem ersten Zeitschritt bestimmt, dann wird das Minutenvolumen für einen zweiten und weitere Zeitschritte konstant gehalten, in denen lediglich eine Atemfrequenz, ein maximaler Inspirationsdruck und/oder ein Tidalvolumen angepasst werden.

Die Regelung des PaCO2 beruht vorzugsweise auf dem Vergleich eines durch ärztliches Personal vorgegebenen individuellen PaCO2-Ziels mit einer Schätzung, PaĈO2, welcher nach der Gleichung PaĈO2=C1 * PetCO2 + C2 linear aus dem endtidalen CO2-Partialdruck PetCO2 im Atemgas ermittelt werden kann. Bevorzugt ist PetCO2 dabei nichtinvasiv über einen CO2-Sensor, bevorzugt am Mundstück, des Patienten messbar und die genannte Gleichung in ihren Parametern C1 und C2 bevorzugt durch das ärztliche Personal während der Laufzeit des Algorithmus anpassbar.

Der aus eine Vergleich der genannten Werte resultierende Fehler e wird zunächst vorzugsweise von einem adaptiven Deadzone-Element (Totzone) gefiltert, bevor er durch einen geeigneten Regler, zum Beispiel einen PI-Regler, wobei P und I variabel eingestellt werden können, in das MV-Ziel umgesetzt werden kann. Das Deadzone-Element ermöglicht vor allem eine schonendere Beatmung durch Reduktion der Anzahl an Stelleingriffen in der Stationarität bei gleichzeitig unbeeinflusstem Einpendeln des Reglers um den Zielwert in der Transienz. Dies wird erreicht, indem vorzugsweise das Deadzone-Element genau dann einen gefilterten Fehler *e̅* = 0 ausgibt, wenn sich die Abweichung zwischen Zielwert und Schätzung e für eine definierte Zeit T innerhalb eines durch den Nutzer vorgegebenen Zielbereichs der Breite ±w um den Zielwert befunden hat. Das Deadzone-Element gibt vorzugsweise *e̅*=*e* aus bevor die o.g. Bedingung zum ersten Mal erfüllt ist, sowie nachdem der absolute Fehler einmalig größer als ein durch den Nutzer setzbarer Parameter L, also |*e*|>*L*, war. Bevorzugt kann der vorgegebene Zielbereich auf 4 mmHg eingestellt werden.

Das vom äußeren Regelkreis vorgegebene MV-Ziel wird bevorzugt im inneren Regelkreis durch einen Ventilationsregler in konkrete Werte für RR und PIP umgesetzt, wobei der Nutzer aus verschiedenen lungenschonenden Modi, bevorzugt ein bis drei Modi, besonders bevorzugt drei Modi, des Ventilationsreglers wählen kann. In allen drei Modi werden RR und PIP bevorzugt in Schritten von 2/min bzw. 1 mbar angepasst und die oberen und unteren Grenzen bevorzugt für RR und PIP durch den Nutzer vorgegeben. In einem ersten Modus "Basic" wird durch den Nutzer zusätzlich ein angestrebter oberer Wert für den PIP, PIPprio, gesetzt. In den ersten Modus verstellt der Regler priorisiert RR, um MV zu erreichen. D.h., dass zunächst der Stellbereich von RR ausgeschöpft wird, bevor PIP verstellt wird. Eine Ausnahme bildet der Fall, wenn PIP über PIPprio liegt und das bereitgestellte MV reduziert werden soll - in diesem Fall wird zunächst PIP auf PIPprio abgesenkt, bevor RR verstellt wird. In einem zweiten Modus "Volume-Target" wird zusätzlich die vom Beatmungsgerät gemessene Compliance Crs des Patienten verwendet. Der Nutzer kann hier einen Zielbereich für das Tidalvolumen Vt angeben. In Abhängigkeit von der Compliance des Patienten und des manuell vom ärztlichen Personal gesetzten Positive End-Expiratory Pressure PEEP stellt der Ventilationsregler RR und PIP so ein, dass das vorgegebene Minutenvolumen priorisiert mit dem gewünschten Vt bereitgestellt wird: MV = RR * (PIP - PEEP) = RR * Vt.

Zu Vergleichszwecken kann der Ventilationsregler einen dritten bereits bekannten Modus zur Minimierung der Atemarbeit umfassen, welcher zum Beispiel für ein gegebenes MV abhängig von Compliance Crs, Resistance R und Dead Space Vd eine optimale Atemfrequenz anstrebt.

Die Erfindung umfasst bevorzugt mindestens eins der folgenden Merkmale: (i) Nutzung des PaCO2 als Regelgröße basierend auf Schätzung PaĈO2, (ii) Nutzung der linearen Schätzfunktion nach obiger Gleichung, wobei C1 und C2 während der Laufzeit angepasst werden können, (iii) adaptives Deadzone-Element mit den o.g. genannten Einstellmöglichkeiten, (iv) Ventilationsregler-Modus "Basic", (v) Ventilationsregler-Modus "Volume-Target". Vorzugsweise sind so Beatmungsparameter aus verschiedenen Funktionen möglich.

Die Erfindung stellt vorzugsweise eine Inter-breath-Steuerung bereit. Weiter bevorzugt wird eine Gesamtmenge der Beatmung angesteuert, wobei eine Beatmungs-Waveform dem Beatmungsgerät überlassen wird.

Die Erfindung betrifft ein Beatmungsgerät und eine Steuereinrichtung zur Ansteuerung einer Atemgasquelle eines Beatmungsgeräts mit einem Ventilator, insbesondere für Neugeborene, wobei die Steuereinrichtung umfasst: (i) eine Zielwertbereitstellungseinheit, die ausgestaltet ist, einen Zielwert des arteriellen CO2-Partialdrucks bereitzustellen, (ii) eine Minutenvolumenermittlungseinheit, die ausgestaltet ist, basierend auf dem Zielwert des arteriellen CO2-Partialdrucks und einem ermittelten Wert oder einem aus dem endtidalen CO2-Partialdruck abgeleiteten Wert für den arteriellen CO2-Partialdruck einen Zielwert eines Minutenvolumens zu ermitteln, falls der ermittelte Wert oder der aus dem endtidalen CO2-Partialdruck abgeleitete Wert für den arteriellen CO2-Partialdruck außerhalb eines ersten vordefinierten Wertebereichs um den Zielwert des arteriellen CO2-Partialdrucks liegt oder über einen Zeitraum kleiner als einem vordefinierten Zeitraum in dem ersten vordefinierten Wertebereich liegt, und (iii) eine Atemgasquellesteuereinheit, die ausgestaltet ist, den Zielwert des Minutenvolumens zu empfangen und den Ventilator basierend auf dem Zielwert des Minutenvolumens anzusteuern. Das erfindungsgemäße Beatmungsgerät erlaubt eine besonders schonende Beatmung.

## Patentansprüche

1. Beatmungsgerät (100, 600) zur Atemgasversorgung, insbesondere von Neugeborenen, umfassend
eine Atemgasquelle (110),
eine Steuereinrichtung (120, 610) zur Ansteuerung der Atemgasquelle (110),
eine mit der Steuereinrichtung (120, 610) verbundene Sensoreinrichtung (130, 640) zum Erfassen eines endtidalen CO2-Partialdrucks (PetCO2),
einen auswechselbaren Atemgasschlauch (150) mit zumindest einem ersten Anschlussstutzen (151) für den Atemgasschlauch (150) und einem zweiten Anschlussstutzen (152) für den Atemgasschlauch (150) an einem Patienteninterface (140),
und eine Nutzerschnittstelle (160), die ausgestaltet ist, Nutzereingaben zu empfangen,
wobei die Steuereinrichtung (120, 610) umfasst:
- eine Zielwertbereitstellungseinheit (121), die ausgestaltet ist, einen Zielwert des arteriellen CO2-Partialdrucks (PaCO2) bereitzustellen,
- eine Minutenvolumenermittlungseinheit (122), die ausgestaltet ist, basierend auf dem Zielwert des arteriellen CO2-Partialdrucks (PaCO2) und einem ermittelten Wert für den arteriellen CO2-Partialdruck und/oder einem aus dem endtidalen CO2-Partialdruck (PetCO2) abgeleiteten Wert für den arteriellen CO2-Partialdruck (Pa^CO2) einen Zielwert eines Minutenvolumens zu ermitteln, falls der ermittelte Wert für den arteriellen CO2-Partialdruck oder der aus dem endtidalen CO2-Partialdruck (PetCO2) abgeleitete Wert für den arteriellen CO2-Partialdruck (Pa^CO2) außerhalb eines ersten vordefinierten Wertebereichs (2w) um den Zielwert des arteriellen CO2-Partialdrucks (PaCO2) liegt oder über einen Zeitraum kleiner als einem vordefinierten Zeitraum (T) in dem ersten vordefinierten Wertebereich (2w) liegt, und
- eine Atemgasquellesteuereinheit (123), die ausgestaltet ist, den Zielwert des Minutenvolumens zu empfangen und die Atemgasquelle basierend auf dem Zielwert des Minutenvolumens anzusteuern.

2. Beatmungsgerät (100, 600) nach Anspruch 1, wobei die Atemgasquellesteuereinheit (123) ferner ausgestaltet ist, einen voreingestellten Wert für das Minutenvolumen zu empfangen und die Atemgasquelle (110) basierend auf dem voreingestellten Wert des Minutenvolumens anzusteuern, falls der ermittelte arterielle CO2-Partialdruck oder der aus dem endtidalen CO2-Partialdruck (PetCO2) abgeleitete Wert für den arteriellen CO2-Partialdruck (Pa^CO2) über den vordefinierten Zeitraum (T) in dem ersten vordefinierten Wertebereich (2w) und nach dem vordefinierten Zeitraum (T) in einem zweiten vordefinierten Wertebereich (2L) um den Zielwert des arteriellen CO2-Partialdrucks (PaCO2) liegt, wobei der zweite vordefinierte Wertebereich (2L) größer ist als der erste vordefinierte Wertebereich (2w).

3. Beatmungsgerät (100, 600) nach einem der vorstehenden Ansprüche, wobei die Atemgasquellesteuereinheit (123) ausgestaltet ist, basierend auf dem ermittelten Zielwert des Minutenvolumens einen ersten maximalen Inspirationsdruck (PIP) und eine erste Atemfrequenz (RR) zum Ansteuern de Atemgasquelle (110) zu ermitteln, und die Atemgasquelle (110) basierend auf dem ersten ermittelten maximalen Inspirationsdruck (PIP) und der ersten ermittelten Atemfrequenz (RR) anzusteuern.

4. Beatmungsgerät (100, 600) nach Anspruch 3, wobei die Atemgasquellesteuereinheit (123) ferner ausgestaltet ist, ein gemessenes Minutenvolumen, den ersten ermittelten maximalen Inspirationsdruck (PIP) und/oder die erste ermittelte Atemfrequenz (RR) zu empfangen, basierend zumindest auf dem gemessenen Minutenvolumen, dem ersten ermittelten maximalen Inspirationsdruck (PIP) und/oder der ersten ermittelten Atemfrequenz (RR) einen zweiten maximalen Inspirationsdruck (PIP) und eine zweite Atemfrequenz (RR) zu ermitteln, und die Atemgasquelle (110) basierend auf dem zweiten ermittelten maximalen Inspirationsdruck (PIP) und der zweiten ermittelten Atemfrequenz (RR) anzusteuern.

5. Beatmungsgerät (100, 600) nach einem der Ansprüche 3 oder 4, wobei die Atemgasquellesteuereinheit (123) ferner ausgestaltet ist, einen PIP-Wertebereich mit einem unteren PIP-Grenzwert und einem oberen PIP-Grenzwert für den maximalen Inspirationsdruck (PIP) und einen RR-Wertebereich mit einem unteren RR-Grenzwert und einem oberen RR-Grenzwert für die Atemfrequenz (RR) zu erhalten, und die Atemgasquelle (110) basierend auf dem ermittelten maximalen Inspirationsdruck (PIP) und der ermittelten Atemfrequenz (RR) anzusteuern, falls der ermittelte maximale Inspirationsdruck (PIP) in dem PIP-Wertebereich und die ermittelte Atemfrequenz (RR) in dem RR-Wertebereich liegt.

6. Beatmungsgerät (100, 600) nach einem der Ansprüche 3 bis 5, wobei die Atemgasquellesteuereinheit (123) ferner ausgestaltet ist, einen Solltidalvolumenbereich Vt mit einem unteren Vt-Sollgrenzwert und einem oberen Vt-Sollgrenzwert zu empfangen, basierend zumindest auf dem ermittelten Minutenvolumen und dem Solltidalvolumenbereich Vt, einen tidalvolumenabhängigen maximalen Inspirationsdruck (PIP) und eine tidalvolumenabhängige Atemfrequenz (RR) zu ermitteln, und die Atemgasquelle (110) basierend auf dem tidalvolumenabhängigen maximalen Inspirationsdruck (PIP) und der tidalvolumenabhängigen Atemfrequenz (RR) anzusteuern.

7. Beatmungsgerät (100, 600) nach Anspruch 6, wobei die Atemgasquellesteuereinheit (123) ferner ausgestaltet ist, falls ein gemessenes Tidalvolumen nicht im Solltidalvolumenbereich Vt liegt, basierend zumindest auf dem ermittelten Minutenvolumen einen zweiten tidalvolumenabhängigen maximalen Inspirationsdruck (PIP2) zu ermitteln und die Atemgasquelle (110) basierend auf dem zweiten tidalvolumenabhängigen maximalen Inspirationsdruck (PIP2) anzusteuern.

8. Beatmungsgerät (100, 600) nach einem der Ansprüche 3 bis 7, wobei die Steuereinrichtung (120, 610) mindestens einen vordefinierten Modus hat, der durch die Nutzerschnittstelle (160) auswählbar ist, wobei die Atemgasquellesteuereinheit (123) ausgestaltet ist, die Atemgasquelle (110) basierend auf dem Zielwert des Minutenvolumens unter Verwendung des vordefinierten Modus anzusteuern,
wobei die Atemgasquellesteuereinheit (123) in einem ersten Modus ferner ausgestaltet ist, einen PIP-Wertebereich mit einem unteren PIP-Grenzwert und einem oberen PIP-Grenzwert für den maximalen Inspirationsdruck (PIP) und einen RR-Wertebereich mit einem unteren RR-Grenzwert und einem oberen RR-Grenzwert für die Atemfrequenz (RR) zu erhalten, einen vordefinierten Wert für den maximalen Inspirationsdruck (PIPprio) zu erhalten und die Atemgasquelle (110) basierend auf der ermittelten Atemfrequenz (RR) anzusteuern, falls der ermittelte Wert für den maximalen Inspirationsdruck (PIP) kleiner ist als der vordefinierte Wert für den maximalen Inspirationsdruck (PIPprio), wobei die ermittelte Atemfrequenz (RR) innerhalb des RR-Wertebereichs liegt.

9. Beatmungsgerät (100, 600) nach Anspruch 8, wobei die Atemgasquellesteuereinheit (123) ferner ausgestaltet ist,
die Atemgasquelle (110) basierend auf dem ermittelten maximalen Inspirationsdruck (PIP) und der ermittelten Atemfrequenz (RR) anzusteuern, falls die ermittelte Atemfrequenz (RR) einem oberen RR-Grenzwert des RR-Wertebereichs entspricht und/oder
die Atemgasquelle (110) basierend auf dem ermittelten maximalen Inspirationsdruck (PIP) und der ermittelten Atemfrequenz (RR) anzusteuern, falls der ermittelte Wert für den maximalen Inspirationsdruck (PIP) größer ist als der vordefinierte Wert für den maximalen Inspirationsdruck (PIPprio).

10. Beatmungsgerät (100, 600) nach Anspruch 9, wobei die Atemgasquellesteuereinheit (123) ferner ausgestaltet ist, falls der ermittelte maximale Inspirationsdruck (PIP) dem vordefinierten Wert für den maximalen Inspirationsdruck (PIPprio) entspricht, die Atemgasquelle (110) basierend auf der ermittelten Atemfrequenz (RR) anzusteuern, wobei die ermittelte Atemfrequenz (RR) innerhalb des RR-Wertebereichs liegt, und, falls die ermittelte Atemfrequenz (RR) einem unteren RR-Grenzwert des RR-Wertebereichs entspricht, die Atemgasquelle (110) basierend auf dem ermittelten maximalen Inspirationsdruck (PIP) anzusteuern.

11. Beatmungsgerät (100, 600) nach einem der Ansprüche 8 bis 10, wobei die Atemgasquellesteuereinheit (123) in einem zweiten Modus ferner ausgestaltet ist, einen PIP-Wertebereich mit einem unteren PIP-Grenzwert und einem oberen PIP-Grenzwert für den maximalen Inspirationsdruck (PIP), einen RR-Wertebereich mit einem unteren RR-Grenzwert und einem oberen RR-Grenzwert für die Atemfrequenz (RR) und ein Solltidalvolumenbereich (Vt) mit einem unteren Vt-Sollgrenzwert und einem oberen Vt-Sollgrenzwert zu erhalten, basierend auf zumindest dem PIP-Wertebereich, dem RR-Wertebereich und dem Solltidalvolumenbereich (Vt) einen maximalen Inspirationsdruck (PIP) zu ermitteln, und die Atemgasquelle (110) basierend auf dem ermittelten maximalen Inspirationsdruck (PIP) anzusteuern, falls ein ermittelter Wert für das Tidalvolumen nicht im Solltidalvolumenbereich (Vt) liegt, basierend auf zumindest dem PIP-Wertebereich, dem RR-Wertebereich und dem Solltidalvolumenbereich (Vt) eine Atemfrequenz (RR) zu ermitteln, und die Atemgasquelle (110) basierend auf der ermittelten Atemfrequenz (RR) anzusteuern, falls der ermittelte Wert für das Tidalvolumen innerhalb des Solltidalvolumenbereichs (Vt) liegt.

12. Beatmungsgerät (100, 600) nach Anspruch 11, wobei die Atemgasquellesteuereinheit (123) ferner ausgestaltet ist, die Atemgasquelle (110) basierend auf dem ermittelten maximalen Inspirationsdruck (PIP) und der ermittelten Atemfrequenz (RR) anzusteuern, falls der ermittelte Wert für das Tidalvolumen nicht im Solltidalvolumenbereich (Vt) liegt und der maximale Inspirationsdruck (PIP) einem unteren oder oberen PIP-Grenzwert des PIP-Wertebereichs entspricht.

13. Steuereinrichtung (120, 610) zur Ansteuerung einer Atemgasquelle eines Beatmungsgeräts (100, 600), insbesondere für Neugeborene, wobei die Steuereinrichtung (120, 610) umfasst:
- eine Zielwertbereitstellungseinheit (121), die ausgestaltet ist, einen Zielwert des arteriellen CO2-Partialdrucks (PaCO2) bereitzustellen,
- eine Minutenvolumenermittlungseinheit (122), die ausgestaltet ist, basierend auf dem Zielwert des arteriellen CO2-Partialdrucks (PaCO2) und einem ermittelten Wert für den arteriellen CO2-Partialdruck und/oder einem aus dem endtidalen CO2-Partialdruck (PetCO2) abgeleiteten Wert für den arteriellen CO2-Partialdruck (Pa^CO2) einen Zielwert eines Minutenvolumens zu ermitteln, falls der ermittelte Wert für den arteriellen CO2-Partialdruck oder der aus dem endtidalen CO2-Partialdruck (PetCO2) abgeleitete Wert für den arteriellen CO2-Partialdruck (Pa^CO2) außerhalb eines ersten vordefinierten Wertebereichs (2w) um den Zielwert des arteriellen CO2-Partialdrucks (PaCO2) liegt oder über einen Zeitraum kleiner als einem vordefinierten Zeitraum (T) in dem ersten vordefinierten Wertebereich (2w) liegt, und
- eine Atemgasquellesteuereinheit (123), die ausgestaltet ist, den Zielwert des Minutenvolumens zu empfangen und die Atemgasquelle (110) basierend auf dem Zielwert des Minutenvolumens anzusteuern.

14. Verfahren (700, 990) zur Steuerung eines Beatmungsgeräts, vorzugsweise ein Beatmungsgerät nach einem der Ansprüche 1 bis 12, umfassend:
Erfassen (991) eines endtidalen CO2-Partialdrucks (PetCO2),
Bereitstellen (992) eines Zielwerts des arteriellen CO2-Partialdrucks (PaCO2),
Ermitteln (993), basierend auf dem Zielwert des arteriellen CO2-Partialdrucks (PaCO2) und einem ermittelten Wert für den arteriellen CO2-Partialdruck und/oder einem aus dem endtidalen CO2-Partialdruck (PetCO2) abgeleiteten Wert für den arteriellen CO2-Partialdruck (Pa^CO2), eines Zielwerts eines Minutenvolumens, falls der ermittelte Wert für den arteriellen CO2-Partialdruck oder der aus dem endtidalen CO2-Partialdruck (PetCO2) abgeleitete Wert für den arteriellen CO2-Partialdruck (Pa^CO2) außerhalb eines ersten vordefinierten Wertebereichs (2w) um den Zielwert des arteriellen CO2-Partialdrucks (PaCO2) liegt und/oder über einen Zeitraum kleiner als einem vordefinierten Zeitraum (T) in dem ersten vordefinierten Wertebereich (2w) liegt, und
Empfangen (994) des Zielwerts des Minutenvolumens und
Ansteuern (995) der Atemgasquelle (110) basierend auf dem Zielwert des Minutenvolumens.

15. Computerprogramm mit Programmmitteln, die eine Steuereinrichtung (120, 610) nach Anspruch 13 dazu veranlassen, die Schritte des Verfahrens (700, 990) nach Anspruch 14 auszuführen, wenn das Computerprogramm auf der Steuereinrichtung (120, 610) ausgeführt wird.

## Claims

1. A ventilator (100, 600) for supplying respiratory gas, in particular to newborns, comprising
a respiratory gas source (110),
a control device (120, 610) for controlling the respiratory gas source (110),
a sensor device (130, 640) connected to the control device (120, 610) for detecting an end- tidal CO2 partial pressure (PetCO2),
an exchangeable respiratory gas hose (150) with at least a first connecting piece (151) for the respiratory gas hose (150) and a second connecting piece (152) for the respiratory gas hose (150) on a patient interface (140),
and a user interface (160) which is configured to receive user input,
wherein the control device (120, 610) comprises:
a target value providing unit (121) which is configured to provide a target value of the arterial CO2 partial pressure (PaCO2),
a minute volume determination unit (122) which is configured to determine a target value of a minute volume based on the target value of the arterial CO2 partial pressure (PaCO2) and a determined value for the arterial CO2 partial pressure and/or a value for the arterial CO2 partial pressure (Pa^CO2) derived from the end-tidal CO2 partial pressure (PetCO2) if the determined value for the arterial CO2 partial pressure or the value for the arterial CO2 partial pressure (Pa^CO2) derived from the end-tidal CO2 partial pressure (PetCO2) lies outside a first predefined value range (2w) around the target value of the arterial CO2 partial pressure (PaCO2) or lies within the first predefined value range (2w) for a time period less than a predefined time period (T), and
a respiratory gas source control unit (123) which is configured to receive the target value of the minute volume and to control the respiratory gas source based on the target value of the minute volume.

2. The ventilator (100, 600) according to claim 1, wherein the respiratory gas source control unit (123) is further configured to receive a preset value for the minute volume and to control the respiratory gas source (110) based on the preset value of the minute volume if the determined arterial CO2 partial pressure or the value for the arterial CO2 partial pressure (Pa^CO2) derived from the end-tidal CO2 partial pressure (PetCO2) lies within the first predefined value range (2w) for the predefined time period (T) and in a second predefined value range (2L) around the target value of the arterial CO2 partial pressure (PaCO2) after the predefined time period (T), wherein the second predefined value range (2L) is greater than the first predefined value range (2w).

3. The ventilator (100, 600) according to any one of the preceding claims, wherein the respiratory gas source control unit (123) is configured to determine a first maximum inspiratory pressure (PIP) and a first respiratory rate (RR) for controlling the respiratory gas source (110) based on the determined target value of the minute volume, and to control the respiratory gas source (110) based on the first determined maximum inspiratory pressure (PIP) and the first determined respiratory rate (RR).

4. The ventilator (100, 600) according to claim 3, wherein the respiratory gas source control unit (123) is further configured to receive a measured minute volume, the first determined maximum inspiratory pressure (PIP) and/or the first determined respiratory rate (RR), to determine a second maximum inspiratory pressure (PIP) and a second respiratory rate (RR) based at least on the measured minute volume, the first determined maximum inspiratory pressure (PIP) and/or the first determined respiratory rate (RR), and to control the respiratory gas source (110) based on the second determined maximum inspiratory pressure (PIP) and the second determined respiratory rate (RR).

5. The ventilator (100, 600) according to any one of claims 3 or 4, wherein the respiratory gas source control unit (123) is further configured to obtain a PIP value range having a lower PIP limit value and an upper PIP limit value for the maximum inspiratory pressure (PIP) and an RR value range having a lower RR limit value and an upper RR limit value for the respiratory rate (RR), and to control the respiratory gas source (110) based on the determined maximum inspiratory pressure (PIP) and the determined respiratory rate (RR) if the determined maximum inspiratory pressure (PIP) lies within the PIP value range and the determined respiratory rate (RR) lies within the RR value range.

6. The ventilator (100, 600) according to any one of claims 3 to 5, wherein the respiratory gas source control unit (123) is further configured to receive a target tidal volume range Vt having a lower Vt target limit value and an upper Vt target limit value, to determine a tidal volume-dependent maximum inspiratory pressure (PIP) and a tidal volume-dependent respiratory rate (RR) based at least on the determined minute volume and the target tidal volume range Vt, and to control the respiratory gas source (110) based on the tidal volume-dependent maximum inspiratory pressure (PIP) and the tidal volume-dependent respiratory rate (RR).

7. The ventilator (100, 600) according to claim 6, wherein the respiratory gas source control unit (123) is further configured, if a measured tidal volume is not within the target tidal volume range Vt, to determine a second tidal volume-dependent maximum inspiratory pressure (PIP2) based at least on the determined minute volume and to control the respiratory gas source (110) based on the second tidal volume-dependent maximum inspiratory pressure (PIP2).

8. The ventilator (100, 600) according to any one of claims 3 to 7, wherein the control device (120, 610) has at least one predefined mode selectable by the user interface (160), wherein the respiratory gas source control unit (123) is configured to control the respiratory gas source (110) based on the target value of the minute volume using the predefined mode,
wherein the respiratory gas source control unit (123) in a first mode is further configured to obtain a PIP value range having a lower PIP limit value and an upper PIP limit value for the maximum inspiratory pressure (PIP) and an RR value range having a lower RR limit value and an upper RR limit value for the respiratory rate (RR), to obtain a predefined value for the maximum inspiratory pressure (PIPprio) and to control the respiratory gas source (110) based on the determined respiratory rate (RR) if the determined value for the maximum inspiratory pressure (PIP) is smaller than the predefined value for the maximum inspiratory pressure (PIPprio), wherein the determined respiratory rate (RR) lies within the RR value range.

9. The ventilator (100, 600) according to claim 8, wherein the respiratory gas source control unit (123) is further configured to
control the respiratory gas source (110) based on the determined maximum inspiratory pressure (PIP) and the determined respiratory rate (RR) if the determined respiratory rate (RR) corresponds to an upper RR limit value of the RR value range and/or
control the respiratory gas source (110) based on the determined maximum inspiratory pressure (PIP) and the determined respiratory rate (RR) if the determined value for the maximum inspiratory pressure (PIP) is greater than the predefined value for the maximum inspiratory pressure (PIPprio).

10. The ventilator (100, 600) according to claim 9, wherein the respiratory gas source control unit (123) is further configured, if the determined maximum inspiratory pressure (PIP) corresponds to the predefined value for the maximum inspiratory pressure (PIPprio), to control the respiratory gas source (110) based on the determined respiratory rate (RR), wherein the determined respiratory rate (RR) lies within the RR value range, and, if the determined respiratory rate (RR) corresponds to a lower RR limit value of the RR value range, to control the respiratory gas source (110) based on the determined maximum inspiratory pressure (PIP).

11. The ventilator (100, 600) according to any one of claims 8 to 10, wherein the respiratory gas source control unit (123) in a second mode is further configured to obtain a PIP value range having a lower PIP limit value and an upper PIP limit value for the maximum inspiratory pressure (PIP), an RR value range having a lower RR limit value and an upper RR limit value for the respiratory rate (RR) and a target tidal volume range (Vt) having a lower Vt target limit value and an upper Vt target limit value, to determine a maximum inspiratory pressure (PIP) based at least on the PIP value range, the RR value range and the target tidal volume range (Vt), and to control the respiratory gas source (110) based on the determined maximum inspiratory pressure (PIP) if a determined value for the tidal volume is not within the target tidal volume range (Vt), to determine a respiratory rate (RR) based at least on the PIP value range, the RR value range and the target tidal volume range (Vt), and to control the respiratory gas source (110) based on the determined respiratory rate (RR) if the determined value for the tidal volume lies within the target tidal volume range (Vt).

12. The ventilator (100, 600) according to claim 11, wherein the respiratory gas source control unit (123) is further configured to control the respiratory gas source (110) based on the determined maximum inspiratory pressure (PIP) and the determined respiratory rate (RR) if the determined value for the tidal volume is not within the target tidal volume range (Vt) and the maximum inspiratory pressure (PIP) corresponds to a lower or upper PIP limit value of the PIP value range.

13. Control device (120, 610) for controlling a respiratory gas source of a ventilator (100, 600), in particular for newborns, wherein the control device (120, 610) comprises:
a target value providing unit (121) which is configured to provide a target value of the arterial CO2 partial pressure (PaCO2),
a minute volume determination unit (122) which is configured to determine a target value of a minute volume based on the target value of the arterial CO2 partial pressure (PaCO2) and a determined value for the arterial CO2 partial pressure and/or a value for the arterial CO2 partial pressure (Pa^CO2) derived from the end-tidal CO2 partial pressure (PetCO2) if the determined value for the arterial CO2 partial pressure or the value for the arterial CO2 partial pressure (Pa^CO2) derived from the end-tidal CO2 partial pressure (PetCO2) lies outside a first predefined value range (2w) around the target value of the arterial CO2 partial pressure (PaCO2) or lies within the first predefined value range (2w) for a time period less than a predefined time period (T), and
a respiratory gas source control unit (123) which is configured to receive the target value of the minute volume and to control the respiratory gas source (110) based on the target value of the minute volume.

14. A method (700, 990) for controlling a ventilator, preferably a ventilator according to any one of claims 1 to 12, comprising:
- Detecting (991) an end-tidal CO2 partial pressure (PetCO2),
- Providing (992) a target value of the arterial CO2 partial pressure (PaCO2),
- Determining (993), based on the target value of the arterial CO2 partial pressure (PaCO2) and a determined value for the arterial CO2 partial pressure and/or a value for the arterial CO2 partial pressure (Pa^CO2) derived from the end-tidal CO2 partial pressure (PetCO2), a target value of a minute volume if the determined value for the arterial CO2 partial pressure or the value for the arterial CO2 partial pressure (Pa^CO2) derived from the end-tidal CO2 partial pressure (PetCO2) lies outside a first predefined value range (2w) around the target value of the arterial CO2 partial pressure (PaCO2) and/or lies within the first predefined value range (2w) for a time period less than a predefined time period (T), and
- Receiving (994) the target value of the minute volume and
- Controlling (995) the respiratory gas source (110) based on the target value of the minute volume.

15. A computer program comprising program means for causing a control device (120, 610) according to claim 13 to perform the steps of the method (700, 990) according to claim 14 when the computer program is executed on the control device (120, 610).

## Revendications

1. Respirateur (100, 600) pour l'alimentation en gaz respiratoire, notamment pour les nouveau-nés, comprenant
une source de gaz respiratoire (110),
un dispositif de commande (120, 610) pour commander la source de gaz respiratoire (110),
un dispositif capteur (130, 640) connecté au dispositif de commande (120, 610) pour détecter une pression partielle de CO2 en fin d'expiration (PetCO2),
un tuyau de gaz respiratoire amovible (150) comprenant au moins un premier raccord (151) pour le tuyau de gaz respiratoire (150) et un second raccord (152) pour le tuyau de gaz respiratoire (150) sur une interface patient (140),
et une interface utilisateur (160) configurée pour recevoir des saisies utilisateur,
dans lequel le dispositif de commande (120, 610) comprend :
une unité de fourniture de valeur cible (121) configurée pour fournir une valeur cible de la pression partielle artérielle de CO2 (PaCO2),
une unité de détermination du volume-minute (122) configurée pour déterminer une valeur cible d'un volume-minute sur la base de la valeur cible de la pression partielle artérielle de CO2 (PaCO2) et d'une valeur déterminée pour la pression partielle artérielle de CO2 et/ou d'une valeur pour la pression partielle artérielle de CO2 (Pa^CO2) dérivée de la pression partielle de CO2 en fin d'expiration (PetCO2), si la valeur déterminée pour la pression partielle artérielle de CO2 ou la valeur pour la pression partielle artérielle de CO2 (Pa^CO2) dérivée de la pression partielle de CO2 en fin d'expiration (PetCO2) est en dehors d'une première plage de valeurs prédéfinie (2w) autour de la valeur cible de la pression partielle artérielle de CO2 (PaCO2) ou se trouve dans la première plage de valeurs prédéfinie (2w) pendant une durée inférieure à une durée prédéfinie (T), et
une unité de commande de la source de gaz respiratoire (123) configurée pour recevoir la valeur cible du volume-minute et pour commander la source de gaz respiratoire sur la base de la valeur cible du volume-minute.

2. Respirateur (100, 600) selon la revendication 1, dans lequel l'unité de commande de la source de gaz respiratoire (123) est en outre configurée pour recevoir une valeur préréglée pour le volume-minute et pour commander la source de gaz respiratoire (110) sur la base de la valeur préréglée du volume-minute, si la pression partielle artérielle de CO2 déterminée ou la valeur pour la pression partielle artérielle de CO2 (Pa^CO2) dérivée de la pression partielle de CO2 en fin d'expiration (PetCO2) se trouve dans la première plage de valeurs prédéfinie (2w) pendant la durée prédéfinie (T) et, après la durée prédéfinie (T), dans une seconde plage de valeurs prédéfinie (2L) autour de la valeur cible de la pression partielle artérielle de CO2 (PaCO2), dans lequel la seconde plage de valeurs prédéfinie (2L) est plus grande que la première plage de valeurs prédéfinie (2w).

3. Respirateur (100, 600) selon l'une quelconque des revendications précédentes, dans lequel l'unité de commande de la source de gaz respiratoire (123) est configurée pour déterminer, sur la base de la valeur cible déterminée du volume-minute, une première pression inspiratoire maximale (PIP) et une première fréquence respiratoire (RR) pour commander la source de gaz respiratoire (110), et pour commander la source de gaz respiratoire (110) sur la base de la première pression inspiratoire maximale déterminée (PIP) et de la première fréquence respiratoire déterminée (RR).

4. Respirateur (100, 600) selon la revendication 3, dans lequel l'unité de commande de la source de gaz respiratoire (123) est en outre configurée pour recevoir un volume-minute mesuré, la première pression inspiratoire maximale déterminée (PIP) et/ou la première fréquence respiratoire déterminée (RR), pour déterminer, sur la base au moins du volume-minute mesuré, de la première pression inspiratoire maximale déterminée (PIP) et/ou de la première fréquence respiratoire déterminée (RR), une seconde pression inspiratoire maximale (PIP) et une seconde fréquence respiratoire (RR), et pour commander la source de gaz respiratoire (110) sur la base de la seconde pression inspiratoire maximale déterminée (PIP) et de la seconde fréquence respiratoire déterminée (RR).

5. Respirateur (100, 600) selon l'une des revendications 3 ou 4, dans lequel l'unité de commande de la source de gaz respiratoire (123) est en outre configurée pour obtenir une plage de valeurs PIP avec une valeur limite PIP inférieure et une valeur limite PIP supérieure pour la pression inspiratoire maximale (PIP) et une plage de valeurs RR avec une valeur limite RR inférieure et une valeur limite RR supérieure pour la fréquence respiratoire (RR), et pour commander la source de gaz respiratoire (110) sur la base de la pression inspiratoire maximale déterminée (PIP) et de la fréquence respiratoire déterminée (RR), si la pression inspiratoire maximale déterminée (PIP) se trouve dans la plage de valeurs PIP et la fréquence respiratoire déterminée (RR) se trouve dans la plage de valeurs RR.

6. Respirateur (100, 600) selon l'une des revendications 3 à 5, dans lequel l'unité de commande de la source de gaz respiratoire (123) est en outre configurée pour recevoir une plage de volume courant souhaitée Vt avec une valeur limite inférieure de Vt souhaitée et une valeur limite supérieure de Vt souhaitée, pour déterminer, sur la base au moins du volume-minute déterminé et de la plage de volume courant souhaitée Vt, une pression inspiratoire maximale dépendant du volume courant (PIP) et une fréquence respiratoire dépendant du volume courant (RR), et pour commander la source de gaz respiratoire (110) sur la base de la pression inspiratoire maximale dépendant du volume courant (PIP) et de la fréquence respiratoire dépendant du volume courant (RR).

7. Respirateur (100, 600) selon la revendication 6, dans lequel l'unité de commande de la source de gaz respiratoire (123) est en outre configurée, si un volume courant mesuré ne se trouve pas dans la plage de volume courant souhaitée Vt, pour déterminer, sur la base au moins du volume-minute déterminé, une seconde pression inspiratoire maximale dépendant du volume courant (PIP2) et pour commander la source de gaz respiratoire (110) sur la base de la seconde pression inspiratoire maximale dépendant du volume courant (PIP2).

8. Respirateur (100, 600) selon l'une des revendications 3 à 7, dans lequel le dispositif de commande (120, 610) dispose d'au moins un mode prédéfini sélectionnable via l'interface utilisateur (160), dans lequel l'unité de commande de la source de gaz respiratoire (123) est configurée pour commander la source de gaz respiratoire (110) sur la base de la valeur cible du volume-minute en utilisant le mode prédéfini,
dans lequel l'unité de commande de la source de gaz respiratoire (123) dans un premier mode est en outre configurée pour obtenir une plage de valeurs PIP avec une valeur limite PIP inférieure et une valeur limite PIP supérieure pour la pression inspiratoire maximale (PIP) et une plage de valeurs RR avec une valeur limite RR inférieure et une valeur limite RR supérieure pour la fréquence respiratoire (RR), pour obtenir une valeur prédéfinie pour la pression inspiratoire maximale (PIPprio) et pour commander la source de gaz respiratoire (110) sur la base de la fréquence respiratoire déterminée (RR), si la valeur déterminée pour la pression inspiratoire maximale (PIP) est inférieure à la valeur prédéfinie pour la pression inspiratoire maximale (PIPprio), dans lequel la fréquence respiratoire déterminée (RR) se trouve dans la plage de valeurs RR.

9. Respirateur (100, 600) selon la revendication 8, dans lequel l'unité de commande de la source de gaz respiratoire (123) est en outre configurée pour
commander la source de gaz respiratoire (110) sur la base de la pression inspiratoire maximale déterminée (PIP) et de la fréquence respiratoire déterminée (RR), si la fréquence respiratoire déterminée (RR) correspond à une valeur limite RR supérieure de la plage de valeurs RR et/ou
commander la source de gaz respiratoire (110) sur la base de la pression inspiratoire maximale déterminée (PIP) et de la fréquence respiratoire déterminée (RR), si la valeur déterminée pour la pression inspiratoire maximale (PIP) est supérieure à la valeur prédéfinie pour la pression inspiratoire maximale (PIPprio).

10. Respirateur (100, 600) selon la revendication 9, dans lequel l'unité de commande de la source de gaz respiratoire (123) est en outre configurée, si la pression inspiratoire maximale déterminée (PIP) correspond à la valeur prédéfinie pour la pression inspiratoire maximale (PIPprio), pour commander la source de gaz respiratoire (110) sur la base de la fréquence respiratoire déterminée (RR), dans lequel la fréquence respiratoire déterminée (RR) se trouve dans la plage de valeurs RR, et, si la fréquence respiratoire déterminée (RR) correspond à une valeur limite RR inférieure de la plage de valeurs RR, pour commander la source de gaz respiratoire (110) sur la base de la pression inspiratoire maximale déterminée (PIP).

11. Respirateur (100, 600) selon l'une des revendications 8 à 10, dans lequel l'unité de commande de la source de gaz respiratoire (123) dans un second mode est en outre configurée pour obtenir une plage de valeurs PIP avec une valeur limite PIP inférieure et une valeur limite PIP supérieure pour la pression inspiratoire maximale (PIP), une plage de valeurs RR avec une valeur limite RR inférieure et une valeur limite RR supérieure pour la fréquence respiratoire (RR) et une plage de volume courant souhaitée (Vt) avec une valeur limite inférieure de Vt souhaitée et une valeur limite supérieure de Vt souhaitée, pour déterminer une pression inspiratoire maximale (PIP) sur la base au moins de la plage de valeurs PIP, de la plage de valeurs RR et de la plage de volume courant souhaitée (Vt), et pour commander la source de gaz respiratoire (110) sur la base de la pression inspiratoire maximale déterminée (PIP), si une valeur déterminée pour le volume courant ne se trouve pas dans la plage de volume courant souhaitée (Vt), pour déterminer une fréquence respiratoire (RR) sur la base au moins de la plage de valeurs PIP, de la plage de valeurs RR et de la plage de volume courant souhaitée (Vt), et pour commander la source de gaz respiratoire (110) sur la base de la fréquence respiratoire déterminée (RR), si la valeur déterminée pour le volume courant se trouve dans la plage de volume courant souhaitée (Vt).

12. Respirateur (100, 600) selon la revendication 11, dans lequel l'unité de commande de la source de gaz respiratoire (123) est en outre configurée pour commander la source de gaz respiratoire (110) sur la base de la pression inspiratoire maximale déterminée (PIP) et de la fréquence respiratoire déterminée (RR), si la valeur déterminée pour le volume courant ne se trouve pas dans la plage de volume courant souhaitée (Vt) et la pression inspiratoire maximale (PIP) correspond à une valeur limite PIP inférieure ou supérieure de la plage de valeurs PIP.

13. Dispositif de commande (120, 610) pour commander une source de gaz respiratoire d'un respirateur (100, 600), notamment pour les nouveau-nés, dans lequel le dispositif de commande (120, 610) comprend :
• une unité de fourniture de valeur cible (121) configurée pour fournir une valeur cible de la pression partielle artérielle de CO2 (PaCO2),
• une unité de détermination du volume-minute (122) configurée pour déterminer une valeur cible d'un volume-minute sur la base de la valeur cible de la pression partielle artérielle de CO2 (PaCO2) et d'une valeur déterminée pour la pression partielle artérielle de CO2 et/ou d'une valeur pour la pression partielle artérielle de CO2 (Pa^CO2) dérivée de la pression partielle de CO2 en fin d'expiration (PetCO2), si la valeur déterminée pour la pression partielle artérielle de CO2 ou la valeur pour la pression partielle artérielle de CO2 (Pa^CO2) dérivée de la pression partielle de CO2 en fin d'expiration (PetCO2) est en dehors d'une première plage de valeurs prédéfinie (2w) autour de la valeur cible de la pression partielle artérielle de CO2 (PaCO2) ou se trouve dans la première plage de valeurs prédéfinie (2w) pendant une durée inférieure à une durée prédéfinie (T), et
• une unité de commande de la source de gaz respiratoire (123) configurée pour recevoir la valeur cible du volume-minute et pour commander la source de gaz respiratoire (110) sur la base de la valeur cible du volume-minute.

14. Procédé (700, 990) de commande d'un respirateur, de préférence un respirateur selon l'une des revendications 1 à 12, comprenant :
la détection (991) d'une pression partielle de CO2 en fin d'expiration (PetCO2),
la fourniture (992) d'une valeur cible de la pression partielle artérielle de CO2 (PaCO2),
la détermination (993), sur la base de la valeur cible de la pression partielle artérielle de CO2 (PaCO2) et d'une valeur déterminée pour la pression partielle artérielle de CO2 et/ou d'une valeur pour la pression partielle artérielle de CO2 (Pa^CO2) dérivée de la pression partielle de CO2 en fin d'expiration (PetCO2), d'une valeur cible d'un volume-minute, si la valeur déterminée pour la pression partielle artérielle de CO2 ou la valeur pour la pression partielle artérielle de CO2 (Pa^CO2) dérivée de la pression partielle de CO2 en fin d'expiration (PetCO2) est en dehors d'une première plage de valeurs prédéfinie (2w) autour de la valeur cible de la pression partielle artérielle de CO2 (PaCO2) et/ou se trouve dans la première plage de valeurs prédéfinie (2w) pendant une durée inférieure à une durée prédéfinie (T), et
la réception (994) de la valeur cible du volume-minute et
la commande (995) de la source de gaz respiratoire (110) sur la base de la valeur cible du volume-minute.

15. Programme informatique comprenant des moyens de programme qui amènent un dispositif de commande (120, 610) selon la revendication 13 à exécuter les étapes du procédé (700, 990) selon la revendication 14, lorsque le programme informatique est exécuté sur le dispositif de commande (120, 610).
